# EUROPEAN PATENT APPLICATION

(11) **EP 2 481 424 A1**
(43) Date of publication of application: **01.08.2012**
(21) Application number: 11180634.5
(22) Date of filing: 20.03.2006
(51) Int. Cl.: A61K 39/395, A61K 39/42, C07K 7/00, C07K 14/18, C07K 16/10

(54) **Improvements in or relating to treatment and prevention of hepatitis C viral infections**

(30) Priority: 19.03.2005 GB 0505697; 23.12.2005 GB 0526421; 23.12.2005 US 315123
(62) Divisional of application: 06726429.1
(71) Applicant: Medical Research Council, Swindon, SN2 1FL (GB)
(72) Inventor: Ball, Jonathan, Nottingham NG7 2UH (GB); Patel, Arvind, Glasgow G11 5JR (GB)
(74) Representative: Smith, Stephen Edward

(57) **Abstract**

Disclosed is polynucleotide encoding a polypeptide comprising an antibody binding site, the polypeptide being able to bind to HCV E2 samples representative of each of HCV genotypes 1-6, as well as polypeptides having such properties and uses of such polypeptides in detecting and treating HCV infection.

## Description

### Field of the Invention

This invention relates to ligands capable of neutralising HCV, various amino acid residue-containing and/or nucleotide-containing compositions for eliciting antibodies against Hepatitis C Virus (HCV), methods for preventing and/or treating HCV infection, and assay apparatus and methods for detecting HCV.

### Background of the Invention

HCV is a positive strand RNA virus belonging to the Flaviviridae family. It is the major cause of non-A non-B viral hepatitis. HCV has infected approximately 200 million people and current estimates suggest that as many as 3 million individuals are newly infected each year (3). Approximately 80% of those infected fail to clear the virus; a chronic infection ensues, frequently leading to severe chronic liver disease, cirrhosis and hepatocellular carcinoma (2, 45). Current treatments for chronic infection are ineffective and there is a pressing need to develop preventative and therapeutic vaccines.

Due to the error-prone nature of the RNA-dependent RNA polymerase and the high replicative rate *in vivo* (34, 50), HCV exhibits a high degree of genetic variability. HCV can be classified into six genetically distinct genotypes and further subdivided into at least 70 subtypes, which differ by approximately 30% and 15% at the nucleotide level, respectively (64, 66). A significant challenge for the development of vaccines will be identifying protective epitopes that are conserved in the majority of viral genotypes and subtypes. This problem is compounded by the fact that the envelope proteins, the natural target for the neutralising response, are two of the most variable proteins (10).

The envelope proteins, E1 and E2, are responsible for cell binding and entry (4, 8, 17, 55, 61). They are N-linked glycosylated (22, 25, 35, 47, 67) transmembrane proteins with an N-terminal ectodomain and a C-terminal hydrophobic membrane anchor (12, 23, 24). *In vitro* expression experiments have shown that E1 and E2 proteins form a non-covalent heterodimer, which is proposed to be the functional complex on the virus surface (14, 15, 18, 24). Due to the lack of an efficient culture system, the exact mechanism of viral entry is unknown. That said, there is mounting evidence that entry into isolated primary liver cells and cell lines requires interaction with the cell surface receptors CD81 and Scavenger Receptor Class B Type 1 (SR-B1) (6, 7, 20, 63, 71), although these receptors alone are not sufficient to allow viral entry.

Current evidence suggests that cell mediated immunity is pivotal in clearance and control of viral replication in acute infection (36, 72). However, surrogate models of infection, such as animal infection and cell and receptor binding assays, have highlighted the potential role of antibodies in both acute and chronic infection (5, 26, 27, 40, 59, 61, 62, 68, 73, 74). Unsurprisingly, neutralising antibodies recognise both linear and conformational epitopes. The majority of antibodies that demonstrate broad neutralisation capacity are directed against conformational epitopes within E2 (1, 9, 37, 38, 40). Induction of antibodies recognising conserved conformational epitopes is extremely relevant to vaccine design, but this is likely to prove difficult, as the variable regions appear to be immuno-dominant (59). One such immuno-dominant linear epitope lies within the first hypervariable region of E2 (HVR1) (73). The use of conserved HVR1 mimotopes has been proposed to overcome problems of restricted specificity (11, 60, 75), but it is not yet known whether this approach will be successful. We, and others, have described that a region immediately downstream of HVR1 contains a number of epitopes (16, 29, 32, 52, 54, 69). One epitope, encompassing residues 412-423 and defined by the monoclonal antibody AP33, inhibits the interaction between CD81 and a range of presentations of E2, including soluble E2, E1E2 and virus-like particles (52).

Whilst AP33 is capable of blocking CD81 binding, it is unknown whether this will directly correlate with neutralisation capacity and, if so, whether or not it will neutralise a diverse range of genetic variants of HCV; an essential property for any promising therapeutic antibody. In addition, it is unknown whether other linear epitopes downstream of HVR1 could also be important in the development of an antibody based vaccine. The inventors (6, 8) and others (71) have recently developed a retroviral pseudo-particle (pp) assay whereby infectivity of the retroviral particles is conferred by HCV E1E2 envelope proteins. This assay can also be used to measure the neutralising capacity of antibodies and sera (5, 44). The inventors describe herein the use of HCVpp reconstituted with E1E2 clones representative of genotypes 1 through to 6 to determine the cross-neutralising capacity of the AP33 antibody and of polyclonal antisera recognising epitopes mapped to a region proximal to the AP33 epitope as well as HVR1.

### Summary of the Invention

The present inventors have surprisingly found that the monoclonal antibody designated AP33, described previously, can bind to and neutralise each of the six known genotypes 1-6 of HCV. The hybridoma secreting the AP33 monoclonal antibody is the subject of a deposit under the Budapest Treaty at the European Collection of Cell Cultures (ECACC, CAMR Porton Down, Salisbury, Wiltshire SP4 OJG UK; date of deposit 27 January 2006, accession number 05122101). Accordingly, it its deduced that the epitope targeted by AP33 is cross-reactive with all of genotypes 1-6 of HCV, indicating it as a target for anti-HCV ligands and as an immunogen for raising anti-HCV antibodies.

In accordance with a first aspect of the invention, therefore, there is provided the use of a ligand capable of binding to an epitope of the HCV E2 polypeptide defined by monoclonal antibody AP33 in the manufacture of a composition for the prophylaxis or treatment of infection by members of each of genotypes 1 to 6 of HCV.

"Defined by", in this context, means that the epitope is the same epitope as is bound by monoclonal antibody AP33, such that the ligand of the invention and AP33 are able to compete for binding to the epitope.

Preferably, the ligand is capable of binding to a polypeptide epitope which has the sequence X₁LX₂NX₃X₄GX₅WX₆X₇, wherein X₁₋₇ is any amino acid.

In a preferred embodiment, X₁ is selected from the group consisting of S, E, Q, H, P and L.

In a preferred embodiment X₂ is selected from the group consisting of V, I, A, R and F.

In a preferred embodiment X₃ is selected form the group consisting of S, T, H, L and A.

In a preferred embodiment X₄ is selected form the group consisting ofN, Q and G.

In a preferred embodiment X₅ is selected form the group consisting of S, K and T.

In a preferred embodiment X₆ is selected form the group consisting of H, R and Q.

In a preferred embodiment X₇ is selected form the group consisting of I, L, F or P.

Advantageously, the polypeptide epitope is selected from the group consisting of QLINTNGSWHI, QLVNTNGSWHI, QLINSNGSWHI, SLINTNGSWHI, ELINTNGSWHI, HLANHQGKWRL, PLFNANGTWQF and ELRNLGGTWRP.

The ligand is preferably an immunoglobulin. As used herein, the term "immunoglobulin" includes members of the immunoglobulin superfamily as described below; preferably, it is an antibody. "Antibody" includes antibody fragments, such as Fab, F(ab')₂, Fv, scFv and single domain antibody (dAb) molecules.

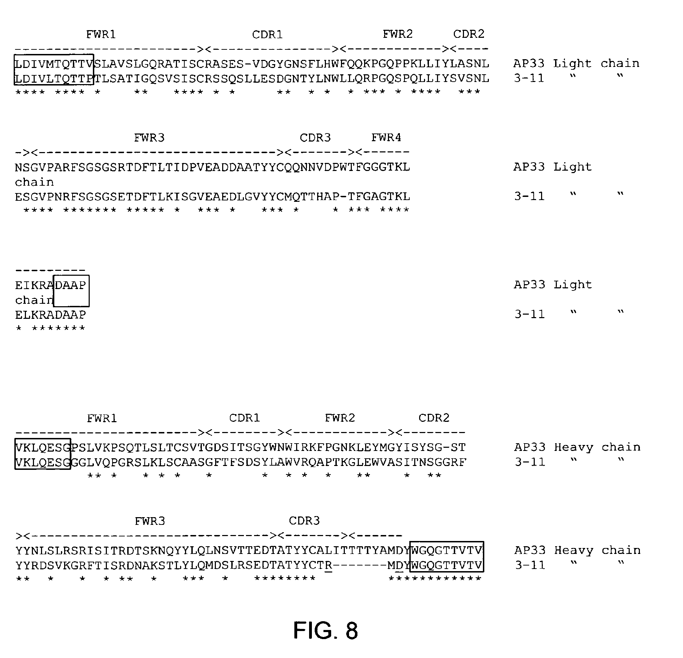

Preferably, the immunoglobulin comprises one or more CDRs derived from monoclonal antibody AP33, as set forth in Figure 8. The CDRs are advantageously selected from the group consisting of:
(a) RASESVDGYGNSFLH, LASNLNS, QQNNVDPWT, GDSITSGYWN, YISYSGSTY or ITTTTYAMDY;
(b) sequences having one, two or three amino acid additions, substitutions or deletions from the sequences set forth in (a); and
(c) sequences structurally similar to the sequences set forth in (a) when present in an immunoglobulin.

Structural similarity, in this case, refers to similarity of the main chain conformation of the resulting polypeptide chain in an immunoglobulin loop. Preferably, structurally similar sequences have a main chain conformation which is with 0.2 Angstrom of the main chain conformation of AP33, and advantageously within 0.1 Angstrom of the main chain conformation of AP33.

Preferably, the invention is useful for the prevention of the infection of a vertebrate cell by HCV. Since the immunoglobulins of the invention are capable of neutralising examples of each of genotypes 1-6 of HCV, the invention is broadly applicable to all HCV infections. Advantageously, the invention allows tests for HCV genotyping to be omitted prior to administration of the ligand, because the immunoglobulin is effective against all HCV genotypes.

In a further aspect, there is provided a method for the prophylaxis or treatment of infection by two or more of genotypes 1-6 of HCV, comprising administering an effective amount of a ligand which binds to an epitope of the HCV E2 polypeptide defined by monoclonal antibody AP33.

In a still further aspect, there is provided a method for the prophylaxis or treatment of infection by two or more of genotypes 1-6 of HCV, comprising administering an effective amount of an immunoglobulin which comprises one or more CDRs derived from monoclonal antibody AP33.

The methods of the invention may comprise features as set forth above in respect of uses of the invention.

The invention moreover provides an immunoglobulin molecule which neutralises HCV isolates belonging to two or more of genotypes 1-6 of HCV, wherein said immunoglobulin comprises one or more CDRs derived from monoclonal antibody AP33, and said immunoglobulin molecule is an immunoglobulin other than the monoclonal antibody AP33.

Advantageously, said one ore more CDRs is selected from the group consisting of:
(a) RASESVDGYGNSFLH, LASNLNS, QQNNVDPWT, GDSITSGYWN, YISYSGSTY or ITTTTYAMDY;
(b) sequences having one, two or three amino acid additions, substitutions or deletions from the sequences set forth in (a); and
(c) sequences structurally similar to the sequences set forth in (a) when present in an immunoglobulin.

In a preferred embodiment, the immunoglobulin is capable of binding to a polypeptide epitope which has the sequence X1LX2NX3X4GX5WX6X7, wherein X1-7 is any amino acid, said immunoglobulin being other than monoclonal antibody AP33.

Preferably, the immunoglobulin comprises one or more human framework regions. Advantageously, it comprises one or more human CDRs. Methods for antibody humanisation and deimmunisation are known in the art, and involve substitution of framework and/or CDR sequences with human sequences, maintaining the specificity of the mouse antibody whilst reducing or eliminating the immunogenicity of the antibody in humans.

### Brief Description of the Figures

Figure 1a shows a series of graphs of absorbance (arbitrary units) against reciprocal of dilution, illustrating the results of various ELISAs;
Figure 1b is a picture showing SDS-PAGE analysis of radiolabelled polypeptides immunoprecipitated by a mixture of the anti-E2 MAbs AP33 and ALP98; numbers on the left hand side indicate molecular weight markers;
Figure 2a is a bar chart showing extent of neutralisation of HCV genotype 1 pseudoparticles by various antisera and pre-immune control sera ("PI" suffix) or the MAb AP33, as measured by % of fluorescent cells (indicating infectivity);
Figure 2b is a similar bar chart showing extent of neutralisation of genotypes 1A, 1B, 2A and 2B HCVpps by various antisera or the MAb AP33;
Figure 2c is a graph of neutralisation (as measured by % of infected cells relative to uninhibited control) against concentration (ng/ml) for the antiserum R646 tested against a variety of genotype 1A HCV subtypes;
Figure 3 is a similar graph, showing neutralisation of different HCV genotypes against concentration of MAb AP33 (in µg/ml);
Figure 4a/b is a representation of part of the amino acid sequence of the E1 protein of various HCV isolates representative of each of the 6 known genotypes.
Figure 5 is a bar chart showing the reactivity of various selected phages, in an E1 Assay, with the antibody AP33 (hollow bar) and ALP98 (solid bar);
Figure 6 shows the deduced amino acid sequence of peptides expressed by various phage clones (Panel A) and their alignment (Panel B) with the corresponding portion of HCV H77 E2 protein.
Figure 7 shows the nucleotide sequence (primer determined sequences omitted) derived by the inventors from the hybridoma which encodes the variable region of the light chain and heavy chain of the AP33 monoclonal antibody;
Figure 8 shows the DNA sequence obtained from cDNA cloned from the AP33 hybridoma (lower case DNA sequence) and the DNA sequence (upper case) of the primers used, for the light and heavy chain variable regions. The deduced amino acid sequence is shown above the DNA sequence. Those amino acid residues constituting the CDRs are shown underlined. An additional residue ('X') is believed to be present at the start of framework region 1 of the heavy chain.
Figure 9 is a bar chart showing the amount of binding of AP33 to various alanine-containing mutants of E1E2 proteins, as judged by EIA, relative to binding to the wild type E1E2 sequence;
Figure 10 is a graph of % binding against antibody concentration, comparing the binding of AP33 (circle symbols) and 3/11 (triangular symbols) to a relevant peptide;
Figure 11 is a series of graphs showing the % binding against antibody concentration (in ng/ml) for AP33 (circles) and 3/11 (triangles) in binding to different E1E2 proteins representative of different HCV genotypes; and
Figure 12 is a bar chart showing % infectivity for various HCVₚₚ clones representing different HCV genotypes, when exposed to AP33 (dark columns) or 3/11 (light columns) at a final concentration of 50 µg/ml. Infectivity is expressed as a percentage of the infectivity of the HCVₚₚ preparation in the absence of MAb.
Figure 13 is a graph showing neutralisation of HCV J71 by AP33 (filled circles) and 3/11 (open circles) monoclonal antibodies. An unrelated monoclonal antibody DB165 (filled triangles) and no antibody (open triangles) are included as controls.

### Detailed Description of the Invention

Unless defined otherwise, all technical and scientific terms used herein have the same meaning as commonly understood by one of ordinary skill in the art (e.g., in cell culture, molecular genetics, nucleic acid chemistry, immunology, antibody engineering and biochemistry). The present invention employs, unless otherwise indicated, conventional techniques which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); Harlow and Lane, Antibodies: a Laboratory Manual, (1988) Cold Spring Harbor. Each of these general texts is herein incorporated by reference.

### A. LIGANDS

A ligand in accordance with the present invention may be any molecule capable of binding to a polypeptide epitope. For example, the ligand may be a protein or nucleic acid aptamer, or an immunoglobulin. Immunoglobulin molecules, according to the present invention, refer to members of the immunoglobulin superfamily, a family of polypeptides which comprise the immunoglobulin fold characteristic of antibody molecules, which contains two β sheets and, usually, a conserved disulphide bond. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signalling (for example, receptor molecules, such as the PDGF receptor). The present invention is applicable to all immunoglobulin superfamily molecules which are capable of binding to target molecules. Preferably, the present invention relates to antibodies. Preferably, a ligand according to the invention neutralises HCV samples representative of each of HCV genotypes 1-6 with an IC₅₀ of 35µg/ml or less, as judged by HCVₚₚ neutralisation assay as described herein.

Conventional antibodies, such as AP33, are large multi-subunit protein molecules comprising at least four polypeptide chains. For example, human IgG has two 'heavy' chains and two 'light' chains that are disulphide-bonded to form a functional antibody. Each heavy and light chain itself comprises a "constant" (C) and a "variable" (V) region. The V regions determine the antigen binding specificity of the antibody, whilst the C regions provide structural support and function in non-antigen-specific interactions with immune effectors.

The antigen binding specificity of an antibody or antigen-binding fragment of an antibody describes the ability of an antibody or fragment thereof to bind to a particular antigen. The antigen binding specificity of an antibody is determined by the structural characteristics of the V region. Each V region typically comprises three complementarity determining regions ("CDRs", each of which contains a "hypervariable loop"), and four framework regions. An antibody binding site, the minimal structural unit required to bind with substantial affinity to a particular desired antigen, will therefore typically include the three CDRs, and at least three, preferably four, framework regions interspersed therebetween to hold and present the CDRs in the appropriate conformation.

Antibodies, as used herein, refers to complete antibodies or antibody fragments capable of binding to a selected target, and including Fv, ScFv, Fab' and F(ab')₂, dAbs, engineered antibodies including chimeric, CDR-grafted and humanised antibodies, and artificially selected antibodies produced using phage display or alternative techniques. Small fragments, such as dAbs, Fv and ScFv, possess advantageous properties for diagnostic and therapeutic applications on account of their small size and consequent superior tissue distribution. Preferably, the antibody is a single chain antibody or scFv.

Generally the antibody will comprise at least three recognisable CDRs or hypervariable loops and at least three, preferably four, recognisable framework regions, and in any event must retain the ability to bind HCV E2 protein. Typically, but not necessarily, the polypeptide will also comprise a light chain constant region and/or a heavy chain constant region, preferably both. The preferred features of the polypeptide will typically be essentially as described above in the context of the polypeptide encoded by the polynucleotide molecule of the first aspect of the invention.

In one particular embodiment the polypeptide may comprise one or more hypervariable loops or CDRs having an amino acid residue sequence substantially or entirely identical to that shown in Figure 8, but comprise one or more framework regions altered so as to correspond to those of a human immunoglobulin. Where the amino acid sequence of the polypeptide diverges from the theoretical ideal of "human frameworks" and "mouse" or "foreign" CDRs, such divergence preferably involves a conservative substitution. A conservative substitution is the substitution of one amino acid residue for another, wherein both residues have a side chain within the same functional group (as defined in Figs. 2.8-2.15 of "Biochemistry" by L. Stryer, 2nd edition W.H. Freeman & Co).

Polypeptides, including non-immunoglobulin polypeptides, having binding activity may be developed, for example, from recombinant libraries of random polypeptide structures. Selection of polypeptides having binding affinity for a desired target by techniques such as phage display, SELEX, mRNA display or surface plasmon resonance, followed if necessary by refinement of the binding specificity and affinity by repeated rounds of mutation and selection, are techniques known to those skilled in the art.

For example, selection of binding polypeptides by mRNA selection is described by Wilson et al., Proc Natl Acad Sci U S A 2001 Mar 27;98(7):3750-3755. Srebalus and Clemmer, Proc Natl Acad Sci U S A 2001 Mar 27;98(7):3750-3755, describe the use of MALDI-TOF MS to characterise the binding of a library of polypeptides to a target molecule. The use of phage display is reviewed by Nilsson et al., Adv Drug Deliv Rev 2000 Sep 30;43(2-3):165-96, and McGregor, Mol Biotechnol 1996 Oct;6(2):155-62. The use of nucleic acid aptamers is reviewed by Hermann and Patel, Science 2000 Feb 4;287(5454):820-5. SELEX is a method for the in vitro evolution of nucleic acid molecules with highly specific binding to target molecules. It is described, for example, in U.S. patents 5654151, 5503978, 5567588 and 5270163, as well as PCT publication WO 96/38579.

Iterative selection procedures such as phage display and SELEX are based on the principle that within a library containing a large number of possible sequences and structures there is a wide range of binding affinities for a given target. A library comprising, for example a 20 subunit randomised polypeptide or nucleic acid polymer can have 4²⁰ structural possibilities. Those which have the higher affinity constants for the target are considered to be most likely to bind. The process of partitioning, dissociation and amplification generates a second nucleic acid library, enriched for the higher binding affinity candidates. Additional rounds of selection progressively favour the best ligands until the resulting library is predominantly composed of only one or a few sequences. These can then be cloned, sequenced and individually tested for binding affinity as pure ligands.

Cycles of selection and mutation/amplification are repeated until a desired goal is achieved. In the most general case, selection/amplification is continued until no significant improvement in binding strength is achieved on repetition of the cycle. The iterative selection/amplification method is sensitive enough to allow isolation of a single sequence variant in a library containing at least 10¹⁴ sequences. The method could, in principle, be used to sample as many as about 10¹⁸ different nucleic acid species. The members of the library preferably include a randomised sequence portion as well as conserved sequences necessary for efficient amplification. Sequence variants can be produced in a number of ways including synthesis of randomised nucleic acid sequences and size selection from randomly cleaved cellular nucleic acids. The variable sequence portion may contain fully or partially random sequence; it may also contain subportions of conserved sequence incorporated with randomised sequence. Sequence variation in test nucleic acids can be introduced or increased by mutagenesis before or during the selection/amplification iterations and by specific modification.

The polypeptide of the invention may be produced from a transformed cell or a transgenic organism using techniques known to those skilled in the art. Typical protocols are provided for illustrative purposes below.

### (a) Transient expression of antibodies in COS-7 cells

DNA can be introduced into COS-7 cells by a number of means such as electroporation, DEAE dextran and calcium phosphate precipitation procedures.

For electroporation, the method of Kettleborough et al (1991 Protein Eng. 4, 773-783) can be used. For co-transfections of heavy and light chain expression vectors 10 µg of each vector is used. For single vectors expressing both antibody chains 13 µg is used. DNA is added to a 0.7 ml aliquot of 1 x 10⁷ cells/ml in PBS and pulsed with 1900 V, 25 µF capacitance using a Bio-Rad Gene Pulser® apparatus. For control purposes 13 µg of vector expressing a non-specific antibody are also transfected into COS-7. COS-7 cells electroporated in the absence of DNA are included as a negative control. Following a 10 min recovery at room temperature, the electroporated cells are added to 8 ml of DMEM containing 5% foetal calf serum (FCS) and incubated for 72 h in 5% CO₂ at 37°C. After 72 h incubation, the medium is collected, spun to remove cellular debris and stored for analysis.

Alternatively, a DEAE-Dextran transfection method can be used. This method is described in Kriegler, M., Gene Transfer and Expression: A Laboratory Manual, W.H Freeman and Company (1990). COS-7 cells are seeded at 1x10⁶ cells/100 mm dish in DMEM (BIOWHITTAKER), 10% foetal bovine serum (FBS). On day two, plasmid DNA is ethanol precipitated, and resuspended at a concentration of 20 µg/ml in sterile TE (10 mM Tris, pH 8.0, 1 mM EDTA). 150 µl of DNA is mixed with 300 µl of sterile TBS (Tris Buffered Saline, 140 mM NaCl, 5 mM KCl, 1.4 mM Na₂HPO₄, 25 mM Tris-base, pH 7.5, 1 mM CaCl₂, and 0.5 mM MgCl₂) and with 300 µl of sterile DEAE dextran (SIGMA, 1 mg/ml in TBS). The growth medium is aspirated, and the cell monolayers are washed once with PBS, and once with TBS. 750 µl of the DNA/DEAE dextran/TBS mixture is added to the monolayer. The dish is incubated at ambient temperature inside a laminar flow hood rocking the dish every 5 min for 1 h. After 1 h incubation, the DNA solution is aspirated and the cells are washed once with TBS and then once with PBS. The cells are incubated in a complete medium supplemented with 100 µM chloroquine (SIGMA), 37°C, 5% CO₂. After 4 h, the medium is replaced with complete medium, and the cells are incubated at 37°C and 5% CO₂. After 48 h post-transfection, the cells are fed with DMEM growth medium lacking serum. 24 h later the medium is harvested, the cell debris removed by centrifugation at 1500 rpm for 5 min in a tabletop clinical centrifuge.

### (b) Stable expression of antibodies of the present invention in CHO cells

A typical protocol for this procedure is as follows:
CHO cells (CHO DUXB-11, Urlaub & Chasin, 1980 Proc. Natl. Acad. Sci. USA 77, 4216-4220) are trypsinized and washed once in phosphate buffered saline (PBS). DNA (13 µg of the plasmid containing the genes for both the heavy and light immunoglobulin chains) and a 0.8 ml aliquot of 1 x 10⁷ cells/ml in PBS are placed in a sterile Gene Pulser® cuvette (0.4 cm gap). A pulse is delivered at 1900 volts, 25 µF capacitance. After a 10 minute recovery period at room temperature, the electroporated cells are added to 20 ml of α-MEM (plus ribonucleosides and deoxyribonucleosides)/10% FBS. After a 24-48 h incubation cells are trypsinized and plated into 100 mm dishes in α-MEM (minus ribonucleosides and deoxyribonucleosides)/10% dialysed FBS (to select for the expression of the dhfr-containing plasmid). Medium is changed every 3-4 days until colonies emerge. Single clones are isolated via cloning cylinders, expanded and analysed for IgG production via ELISA. Single clones are then subjected to increasing concentrations of methotrexate (MTX) in sequential rounds (starting from 10⁹ M⁻¹ MTX) to select for clones expressing increasing amounts of IgG. Medium is changed every 3-4 days until colonies emerge. Single clones are isolated via cloning cylinders, expanded and analysed for IgG production via ELISA.

### (c) Enzymatic production of antibody fragments

Antigen-binding antibody fragments can be produced by enzymatic or chemical separation of intact immunoglobulins. Fragments can also be produced by recombinant DNA techniques (e.g. King et al, 1992 Biochem. J. 281, 317-323; Carter et al, 1992 Biotechnology 10, 163-167). Segments of nucleic acids encoding selected fragments are produced by digestion of full-length coding sequences with relevant restriction enzymes, or by *de novo* synthesis.

For example, a F(ab')₂ fragment can be obtained from an IgG molecule by proteolytic digestion with pepsin at pH 3.0-3.5 using standard methods such as those described in Harlow & Lane (1988 "Antibodies, A Laboratory Manual", Cold Spring Harbor Laboratory, NY).

Fab fragments may be obtained from F(ab')₂ fragments by limited reduction, or from whole antibody by digestion with papain in the presence of reducing agents.

The polypeptides of the present invention may be characterised in a number of ways which will be apparent to those skilled in the art. These include physical measurements of the concentration by techniques such as ELISA, and of the antibody purity by SDS-PAGE. In addition the efficacy of the polypeptides can be determined by detecting the binding of the molecule to HCV E2 glycoprotein in solution or in a solid phase system such as ELISA, surface plasmon resonance (e.g. BIAcore) or immunofluorescence assays. More especially, the neutralising capability of the polypeptide can be tested against HCV samples representative of the six known genotypes in a HCV pp-neutralising assay as described herein.

The polypeptides of the invention may comprise non-amino acid moieties. For example, the polypeptides may be glycosylated. Such glycosylation may occur naturally during expression of the polypeptide in the host cell or host organism, or may be a deliberate modification arising from human intervention. Additionally or alternatively the polypeptides of the invention may be subjected to other chemical modification. One such desirable modification is addition of one or more polyethylene glycol (PEG) moieties. PEGylation has been shown to increase significantly the half-life of various antibody fragments *in vivo* (reviewed by Chapman 2002 Adv. Drug Delivery Rev. 54, 531-545). However, random PEGylation of antibody fragments can have highly detrimental effects on the binding affinity of the fragment for the antigen. In order to avoid this it is desirable that PEGylation is restricted to specific, targeted residues of the antibody or antibody fragment (see Knight et a/, 2004 Platelets 15, 409-418 and Chapman, cited above).

### B. ANTIBODY ENGINEERING

The Antibodies according to the invention are advantageously engineered antibodies, for example such that they have a primary sequence which differs from that sequence of an antibody which occurs in nature. In particular, the AP33 antibody is preferably modified.

Antibodies in accordance with the invention, which do not possess the natural AP33 sequence, may be fragments of AP33, modified AP33 comprising one or more additions, substitutions or deletions in its amino acid sequence, additions of labels or effector groups, or the like. Advantageously, the antibody is humanised or deimmunised in order to render it less immunogenic in human subjects.

Antibodies useful in the present invention may be generated *de novo,* or may be produced by engineering AP33.

### B(i) de novo Antibody generation

Antibodies may be generated by immunisation of animals or humans using peptide immunogens as described herein. Antibodies may be obtained from serum of immunised animals, or produced in cell culture. Recombinant DNA technology may be used to produce the antibodies according to established procedure, in bacterial or preferably mammalian cell culture. The selected cell culture system preferably secretes the antibody product.

The general methodology for making monoclonal antibodies by hybridomas is well known. The production of non-human monoclonal antibodies, e.g. murine, lagomorph, equine, is well known and can be accomplished by, for example, immunising an animal with a preparation containing HCV E2 glycoprotein or fragments thereof. Antibody-producing cells obtained from the immunised animals are immortalised and screened, or screened first for the production of antibody which binds to E2 and then immortalised. (See Harlow & Lane, cited above).

Immortal antibody-producing cell lines can be created by cell fusion, and also by other techniques such as direct transformation of B lymphocytes with oncogenic DNA, or transfection with Epstein-Barr virus. Panels of monoclonal antibodies produced against HCV E2 epitopes can be screened for various properties; e.g. for isotype and epitope affinity.

HCV E2-containing polypeptides can also be used to select for human monoclonal antibodies. Some human antibodies may be selected by competitive binding experiments, for example, to have the same epitope specificity as a particular mouse antibody, such as AP33. Such antibodies are particularly likely to share the useful HCV-neutralising properties demonstrated for AP33. Human antibodies to HCV E2 can be produced by screening a DNA library from human B cells (see Huse et al, 1989 Science 246, 1275-1281). Antibodies binding to HCV E2 or a fragment thereof are selected. Sequences encoding such antibodies (or binding fragments) may then be cloned and amplified. This protocol is improved by combination with phage-display technology (e.g. WO 91/17271 and WO 92/01047).

Multiplication of hybridoma cells or mammalian host cells in vitro is carried out in suitable culture media, which are the customary standard culture media, for example Dulbecco's Modified Eagle Medium (DMEM) or RPMI 1640 medium, optionally replenished by a mammalian serum, e.g. foetal calf serum, or trace elements and growth sustaining supplements, e.g. feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

In vitro production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilised or entrapped cell culture, e.g. in hollow fibres, microcapsules, on agarose microbeads or ceramic cartridges.

Large quantities of the desired antibodies can also be obtained by multiplying mammalian cells in vivo. For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor, incorporated herein by reference. Techniques for the preparation of recombinant antibody molecules is described in the above references and also in, for example, EP 0623679; EP 0368684 and EP 0436597, which are incorporated herein by reference.

The cell culture supernatants are screened for the desired antibodies, preferentially by immunofluorescent staining of cells expressing the desired target by immunoblotting, by an enzyme immunoassay, e.g. a sandwich assay or a dot-assay, or a radioimmunoassay.

For isolation of the antibodies, the immunoglobulins in the culture supernatants or in the ascitic fluid may be concentrated, e.g. by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ion-exchange chromatography, chromatography over DEAE-cellulose and/or (immuno-)affinity chromatography, e.g. affinity chromatography with the target molecule or with Protein-A. Antibodies generated according to the foregoing procedures may be cloned by isolation of nucleic acid from cells, according to standard procedures. Usefully, nucleic acids variable domains of the antibodies may be isolated and used to construct antibody fragments, such as scFv.

Fully human antibodies specific for any desired polypeptide may also be produced by selection from libraries, or in transgenic mice which carry a human antibody gene repertoire.

Various techniques for selection of antibodies from libraries have been described, and are reviewed by Hoogenboom (2005) Nature Biotechnology 23, 1105-1116. Briefly, the methods available for antibody library selection include phage display, ribosome display and microbial cell display.

Any library selection system may be used in conjunction with the invention. Selection protocols for isolating desired members of large libraries are known in the art, as typified by phage display techniques. Such systems, in which diverse peptide sequences are displayed on the surface of filamentous bacteriophage (Scott and Smith (1990 *supra),* have proven useful for creating libraries of antibody fragments (and the nucleotide sequences that encoding them) for the *in vitro* selection and amplification of specific antibody fragments that bind a target antigen. The nucleotide sequences encoding the V_{H} and V_{L} regions are linked to gene fragments which encode leader signals that direct them to the periplasmic space of *E. coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty *et al.* (1990) **supra**; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad. Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al.* (1991) supra; Barbas *et al.* (1992) supra; Hawkins and Winter (1992) J. Immunol., 22: 867; Marks et al., 1992, J. Biol. Chem., 267: 16007; Lerner et al. (1992) Science, 258: 1313, incorporated herein by reference).

One particularly advantageous approach has been the use of scFv phage-libraries (Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al.* (1990) supra; Clackson *et al.* (1991) supra; Marks *et al.* (1991) supra; Chiswell et al. (1992) Trends Biotech., 10: 80; Marks *et al.* (1992) supra). Various embodiments of scFv libraries displayed on bacteriophage coat proteins have been described. Refinements of phage display approaches are also known, for example as described in WO96/06213 and WO92/01047 (Medical Research Council et al.) and WO97/08320 (Morphosys, supra), which are incorporated herein by reference.

In phage display methods, libraries of phage are produced in which members display different antibodies or fragments on their outer surfaces. Antibodies are usually displayed as scFv or Fab fragments. Phage displaying antibodies with a desired specificity are selected by affinity enrichment to HCV E2 polypeptide or a fragment thereof.

In a variation of the phage-display method, human antibodies having the binding specificity of a selected murine MAb, such as AP33, can be produced (see WO 92/20791). In this technique, either the heavy or light chain variable region of the selected murine antibody (e.g. AP33) is used as a starting material. If, for example, a light chain variable region is selected as the starting material, a phage library is constructed in which members display the same light chain variable region (i.e. the murine starting material) and a different heavy chain variable region. The heavy chain variable regions may be obtained from a library of rearranged human heavy chain variable regions. A phage showing strong specific binding for HCV E2 glycoprotein (e.g. at least 10⁸ and preferably at least 10⁹ M⁻¹) is selected. The human heavy chain variable region from this phage then serves as a starting material for constructing a further phage library. In this library, each phage displays the same heavy chain variable region (i.e. the region identified from the first display library) and a different light chain variable region. The light chain variable regions are obtained from a library of rearranged human variable light chain regions. Again, phage showing strong specific binding for HCV E2 are selected. These phage display the variable regions of completely human HCV E2 antibodies. These antibodies usually have the same or similar epitope specificity as the murine starting material. As a variant of this, selection may additionally or alternatively be on the basis of ability to neutralise all six genotypes of HCV.

HCV E2-binding polypeptides of the invention may also be expressed by and purified from transgenic organisms, such as transgenic goat, mouse or plant lines. Production of recombinant antibodies in plants was reviewed by Schillberg et al, (2005 Vaccine 23, 1764-1769 and 2003 Cell Mol. Life Sci. 60, 433-445). Plants used successfully for the expression of antibodies or antibody fragments include *Arabidopsis* (De Wilde et al, 1998 Plant Cell Physiol. 39, 639-646) and tobacco (Valdes et al, 2003 Biochem. Biophys. Res. Comm. 308, 94-100).

Once expressed, the whole antibodies or antibody fragments of the present invention can be purified according to standard procedures of the art, including ammonium sulphate precipitation, affinity columns, column chromatography, gel electrophoresis and the like (see generally Scopies & Stoter, 1982 Methods Enzymol. 90 Part E, 479-490). Substantially pure immunoglobulins of at least about 90 to 95% homogeneity are preferred, and 98 to 99% or more homogeneity most preferred, for pharmaceutical uses.

Alternative library selection technologies include bacteriophage lambda expression systems, which may be screened directly as bacteriophage plaques or as colonies of lysogens, both as previously described (Huse et al. (1989) Science, 246: 1275; Caton and Koprowski (1990) Proc. Natl. Acad. Sci. U.S.A., 87; Mullinax et al. (1990) Proc. Natl. Acad Sci. US.A., 87: 8095; Persson et al. (1991) Proc. Natl. Acad. Sci. U.S.A., 88: 2432) and are of use in the invention. Whilst such expression systems can be used to screening up to 10⁶ different members of a library, they are not really suited to screening of larger numbers (greater than 10⁶ members). Other screening systems rely, for example, on direct chemical synthesis of library members. One early method involves the synthesis of peptides on a set of pins or rods, such as described in WO84/03564. A similar method involving peptide synthesis on beads, which forms a peptide library in which each bead is an individual library member, is described in U.S. Patent No. 4,631,211 and a related method is described in WO92/00091. A significant improvement of the bead-based methods involves tagging each bead with a unique identifier tag, such as an oligonucleotide, so as to facilitate identification of the amino acid sequence of each library member. These improved bead-based methods are described in WO93/06121.

Another chemical synthesis method involves the synthesis of arrays of peptides (or peptidomimetics) on a surface in a manner that places each distinct library member (e.g., unique peptide sequence) at a discrete, predefined location in the array. The identity of each library member is determined by its spatial location in the array. The locations in the array where binding interactions between a predetermined molecule (e.g., a receptor) and reactive library members occur is determined, thereby identifying the sequences of the reactive library members on the basis of spatial location. These methods are described in U.S. Patent No. 5,143,854; WO90/15070 and WO92/10092 Fodor et al. (1991) Science, 251: 767; Dower and Fodor (1991) Ann. Rep. Med. Chem., 26: 271.

Other systems for generating libraries of polypeptides or nucleotides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bach (1990) Nucleic Acids Res., 18: 3203; Beaudry and Joyce (1992) Science, 257: 635; WO92/05258 and WO92/14843). In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection. These and all the foregoing documents also are incorporated herein by reference.

An alternative to the use of phage or other cloned libraries is to use nucleic acid, preferably RNA, derived from the spleen of an animal which has been immunised with the selected target. RNA thus obtained represents a natural library of immunoglobulins. Isolation of V-region and C-region mRNA permits antibody fragments, such as Fab or Fv, to be expressed intracellularly in accordance with the invention.

Briefly, RNA is isolated from the spleen of an immunised animal and PCR primers used to amplify V_{H} and V_{L} cDNA selectively from the RNA pool. The V_{H} and V_{L} sequences thus obtained are joined to make scFv antibodies. PCR primer sequences are based on published V_{H} and V_{L} sequences and are available commercially in kit form.

In conjunction with all selection and display systems, the invention provides peptides which have been identified to form the epitope bound by AP33 for isolation of desired binding activities. Such peptides are described in more detail herein.

### B(ii). Engineering of AP33

AP33, or other antibodies sharing the epitope specificity of AP33, may be engineered to reduce immunogenicity and/or improve binding characteristics.

Several techniques for engineering antibodies are known in the art. Generally, antibodies are rendered less immunogenic by transferring CDRs from a donor (non-human) antibody to an acceptor (human) antibody framework; this procedure is known as CDR grafting, or humanisation. A disadvantage of this procedure is that, as a result of differences between donor and acceptor frameworks, binding activity may be lost. Moreover, a certain amount of immunogenicity may be retained by the CDRs themselves. Various complementary and alternative techniques, including veneering, resurfacing, SDR transfer and deimmunisation have been proposed to address these problems.

Preferably the polynucleotide of the present invention encodes a CDR-grafted molecule. A CDR-grafted molecule is one which comprises light and/or heavy chain CDRs having an amino acid sequence substantially or entirely identical to the CDR sequences of the light or heavy chain of AP33 shown in Figure 8, and framework regions which are not substantially identical to the framework region sequences of AP33 shown in Figure 8. For present purposes, CDRs are considered "substantially identical" to those of AP33 if each CDR sequence differs from that of the corresponding CDR shown in Figure 8 by no more than one two amino acid residues and preferably no more than amino acid residue (i.e. preferably no more than one amino acid residue substitution in each CDR relative to the CDR sequences of AP33 shown in Figure 8). Preferably the CDR sequences of the CDR grafted molecule will be entirely identical to those of AP33 shown in Figure 8.

A particularly preferred type of CDR-grafted molecule is an antibody or antigen-binding fragments thereof comprising CDRs having an amino acid sequence substantially or entirely identical to those of AP33 shown in Figure 8, but human framework region sequences. Such a molecule may be described as "humanised". The use of framework regions altered, relative to those in AP33, so as to be more closely similar (or even identical) to those of a human antibody should greatly reduce the immunogenicity of the resulting polypeptide (relative to AP33) in a human subject A "human framework region sequence" is one which is identical to that of a human antibody or which differs therefrom by an insignificant amount (e.g. by no more than 7 amino acid residues per framework region, preferably no more than 4 residues per framework region, more preferably no more than 3 residues per framework region, and most preferably no more than 2 residues per framework region). Conveniently the encoded polypeptide comprises framework regions identical to those encoded by a human germline antibody gene segment.

In general terms, "CDR-grafting" involves the formation of a polynucleotide which encodes CDRs from a non-human origin (such as a mouse or other non-human mammal) in combination with human framework regions. Constant regions, if present, are preferably also of human origin.

Conventionally the terms "donor antibody" and "acceptor antibody" are used: the CDRs from a non-human donor antibody being grafted into the frameworks of a human acceptor antibody. Techniques of CDR grafting are well-known to those skilled in the art. The procedure was originally described by Jones et al, (1986 Nature 321, 522-525) and by Riechmann et al, (1988 Nature 332, 323-327) and involved the grafting of only CDRs of non-human origin into human frameworks.

This technique can require the changing of certain framework residues, outside of the CDRs, were additionally transferred into the grafted antibody (Riechmann et al, (1988) Nature 332, 323-327). Accordingly, in the present specification the term "CDR-grafting" should not be construed as meaning solely the transfer of CDR residues into a different framework but encompasses also the additional transfer of such framework residues as may be necessary substantially to confer upon the resulting molecule the antigen binding specificity of the antibody from which the CDRs are derived. The term "CDR-grafted molecule" should correspondingly be construed as encompassing polypeptides in which certain framework residues are also "grafted", as well as the CDRs.

Antibody humanisation has been described in, for example, EP460167, EP682040, US5530101, US5585089, US5693761, US5693762, US5766886, US5821337, US5859205, US5886152, US5887293, US5955358, US6054297 and US6180370. These methods all involve redesigning the variable region of an antibody so that the amino acid residues responsible for conferring the antigen binding specificity are integrated into the framework regions of a human antibody variable region.

In some cases the immunogenic portions of a non-human antibody are replaced by residues from a human antibody (e.g. US5712120). Alternatively the residues on the surface of the antibody variable domain can be replaced by residues from a human antibody to "resurface" the non-human variable domain (e.g. US5639641). Resurfacing was suggested by Padlan (1991, EP0519596) and is also termed "veneering". In this procedure the solvent-accessible residues of a first (equivalent of the donor ― source of CDRs) antibody are replaced by residues from a second ("acceptor") antibody. Typically, the second antibody is a human antibody. The solvent inaccessible residues, CDR's, inter-domain contact residues, and residues immediately flanking the CDR's all remain as in the first antibody. This strategy is intended to mimic the surface of a second antibody while retaining all of the packing and interface interactions from the first antibody, which may aid in retention of full antigen binding activity. This should reduce the number of B cell epitopes (and may also reduce some T epitopes), leading to lower immunogenicity.

The solvent accessible residues are identified by inspection of high-resolution structures of antibodies. Other regions of the antibody which may be relevant to humanisation: buried residues which make contact with the CDR's and are different between the murine and human antibodies (in such cases the rodent residue is used); the N-terminal regions which are positioned near the CDR's for both domains and may play a role in antigen binding; electrostatic interactions, which may also play a part even at long distance. The choice of surface residues to be substituted is determined by homology matching between the first antibody variable domains and those of available sequences (either individual or consensus sequences) from the second species.

US5639641 and EP0592106A1 describe alternative methods for resurfacing. Here solvent accessible residues that should be altered to those of a second species are identified using a similar procedure to that of Padlan, but analysing a larger number of structures to obtain average accessibility for each location. Residues that have accessibility above a certain level are examined and are changed for that from an antibody from the species where the antibody is to be used. The choice of residue to be substituted can be from an antibody with overall homology or from the antibody with highest homology taking into consideration only the solvent accessible residues.

A humanisation method described in WO93/17105 and US5766686 identifies low risk residues that can usually safely be altered to the human equivalent. These residues tend to be solvent accessible, Therefore, if only solvent accessible residue are altered, this process would resemble a resurfacing method.

Two further procedures have been described that have the net effect of providing a resurfaced or veneered antibody; see EP0438310A1 and EP0519596A1.

A further technique seeks to identify and remove T cell epitopes (called "detope") so that T help for an immune response is unavailable or reduced, leading to a minimal immune response to the introduced antibody (US5712120; EP0699755A2). It is also possible that B cell epitopes are abolished in this process.

"DeImmunisation" technology seeks to reduce both B and T cell epitopes in an antibody sequence and is dependant on prediction algorithms and also on structural information to model MHC peptide binding sites to identify these motifs. See WO98/52976; EP0983303; WO00/34317; EP1051432).

Antibody humanisation techniques are also taught in "Antibody Engineering" (Eds. Kontermann and Dhubel), Chapter 40 p567-592 (O'Brien and Jones).

Some studies have found that there are examples where not all of the CDRs make direct contact with antigen e.g. MacCallum et al, (1996 J. Mol. Biol. 262, 732-745). Thus, in some instances, humanisation can be achieved by transfer of a subset of CDR residues e.g. Santos & Padlan (1988, Prog. Nucl. Acid Res. Mol. Biol. 60, 169-194) and Tamura et al, (2000, J. Immunol. 164, 1432-1441). This referred to as SDR transfer.

### B(iii). Combined humanisation and selection

Uncertainty in the relative importance of the frameworks has been one factor in the development of the various procedures for successful humanisation and has, in part, led to various selection-driven library procedures. These seek to identify pragmatically the best binders from populations of alternative combinations. For example:
- Use of libraries of specific antibody variable domains with alternate possibilities at one or more framework position to test humanisation variants (e.g. Baca et al. (1997) J. Biol. Chem. 272:10678)
- Use of libraries of variable region frameworks in combination with CDR3s from a specific source (e.g. Rader et al. (1998) PNAS 95:8910)
- Selection of human partner domains from within libraries to substitute for non-human antibody variable domains to form human antibodies or human antibody binding sites of specific affinity and specificity (e.g. Guided selection, Jespers et al. (1994) BioTechnology 12:899; Beiboer et al., (2000) J. Mol. Biol. 296:833).
- Use of specific framework(s) with libraries of CDR3s (e.g. HuCal: Knappick et al., (2000) J. Mol. Biol. 296:57) or all CDRs to obtain specific binding

Wu et al, (1999 J. Mol. Biol. 294, 151-162) have described a procedure for humanisation and simultaneous affinity improvement of the humanised antibody (see also US5955358). A combinatorial library examined eight potentially important framework positions concomitantly with focused CDRH3 and CDRL3 libraries. Multiple anti-CD40 Fab variants containing as few as one murine framework residue and displaying up to approximately 500-fold higher affinity than the initial chimeric Fab were identified.

Phage-display technology offers powerful techniques for selecting such immunoglobulins (see e.g. WO91/17271, WO92/0104 WO92/06204). Thus, for example, humanisation of antibodies may be accomplished using the epitope "imprinting" technique of Hoogenboom and others (e.g. as described by Hoogenboom & Winter 1992, J. Mol. Biol. 227, 381-388; and reviewed by Hoogenboom 2002 Methods Mol. Biol. 178, 1-37 and 2005 Nature Biotechnol. 23, 1105-1116) using phage display and bacterial cells, or using the modified version thereof, employing vaccinia virus display libraries and mammalian cells, developed by Vaccinex Inc. (e.g. as described in US 2005/0266425).

### C: POLYNUCLEOTIDES

The invention provides polynucleotides which encode polypeptide ligands as described herein. The polynucleotide of the present invention may encode any polypeptide which possesses the desired HCV E2-binding activity.

For example, the polynucleotide may encode an entire immunoglobulin molecule chain, such as light chain or a heavy chain. A complete heavy chain includes not only a heavy chain variable region (V_{H}) but also a heavy chain constant region (C_{H}), which typically will comprise three constant domains: C_{H}1, C_{H}2 and C_{H}3; and a "hinge" region. In some situations, the presence of a constant region is desirable. For example, where the antibody is desired to kill an HCV-infected cell, the presence of a complete constant region is desirable to activate complement. However, in other situations the presence of a complete constant region may be undesirable. For instance, where the antibody is required for imaging, tissue penetration may be reduced due to increased molecule size if the constant region is present.

Other polypeptides which may be encoded by the polynucleotide include antigen-binding antibody fragments such as single domain antibodies ("dAbs"), Fv, scFv, Fab' and F(ab')₂ and "minibodies". Minibodies are (typically) bivalent antibody fragments from which the C_{H}1 and C_{K} or C_{L} domain has been excised. As minibodies are smaller than conventional antibodies they should achieve better tissue penetration in clinical/diagnostic use, but being bivalent they should retain higher binding affinity than monovalent antibody fragments, such as dAbs. Accordingly, unless the context dictates otherwise, the term "antibody" as used herein encompasses not only whole antibody molecules but also antigen-binding antibody fragments of the type discussed above.

In a particular embodiment the invention provides a polynucleotide sequence encoding a polypeptide comprising at least three immunoglobulin hypervariable heavy or light chain loops, which polypeptide retains antigen binding and which, when combined with a polypeptide comprising three complementary immunoglobulin hypervariable light or heavy chain loops, forms an antibody molecule or fragment thereof which neutralises HCV samples representative of each of HCV genotypes 1-6 with an IC₅₀ of 35 µg/ml or less, as judged by the HCVpp neutralisation assay described herein.

The hypervariable loops encoded by the polynucleotide may preferably have an amino acid sequence identical or substantially identical to the amino acid sequence of the hypervariable loops present in AP33. The loops are represented by amino acid residues 24-34, 50-56 and 89-87 in the AP33 light chain and 31-35B, 50-65 and 95-102 in the AP33 heavy chain as shown in Figure 8, using the numbering convention devised by Kabat et al, (1991, Sequences of Immunological Interest, 5th Edn. US Dept. Health and Human Services, Washington D.C.).

Whilst the encoded polypeptide will typically have CDR sequences identical or substantially identical to those of AP33, the framework regions will preferably differ from those of AP33. The polynucleotide of the invention will thus preferably encode a polypeptide having a heavy and/or light chain variable region which contains amino acid residue substitutions, especially in the framework regions, relative to the heavy and/or light chain (as appropriate) of AP33. If the encoded polypeptide comprises a partial or complete heavy and/or light chain constant region, this too may comprise substitutions relative to the constant region of AP33.

The effect of the substitutions may be such that, relative to AP33, the encoded polypeptide, has:
(i) increased affinity of binding to HCV E2 protein (e.g. as determined by standard ELISA); and/or
(ii) increased specificity of binding to HCV E2 protein (i.e. reduced cross-reactivity with other proteins, especially human proteins); and/or
(iii) decreased IC₅₀ for neutralisation of one or more genotypes of HCV as determined by the HCVₚₚ neutralisation assay described herein; and/or
(iv) decreased immunogenicity in a human subject, (e.g. as determined by anti-idiotype response measured by standard ELISA, following intravenous administration of a standard dose of the encoded polypeptide to a human subject).

Preferably at least one of the framework regions of the encoded polypeptide, and most preferably each of the framework regions, will comprise amino acid substitutions relative to AP33 so as to become more similar to those of a human antibody, so as to reduce the immunogenicity (relative to AP33) of the resulting polypeptide in a human subject.

Preferably each framework region present in the encoded polypeptide will comprise at least one amino acid substitution relative to the corresponding AP33 framework. Thus, for example, the framework regions may comprise, in total, three, four, five, six, seven, eight, nine, ten, eleven, twelve, thirteen, fourteen or fifteen amino acid substitutions relative to the framework regions present in AP33.

It is possible, that the hypervariable loops of the polypeptide encoded by the polynucleotide may also comprise a total of one or more amino acid substitutions relative to the amino sequence of AP33 as shown in Figure 8. The encoded polypeptide may, for instance, comprise one, two, three, four, five, six, seven, eight, nine, ten, eleven or twelve amino acid substitutions (in the heavy and/or light chain) relative to the AP33 hypervariable loop sequences. Possibly each of the hypervariable loops may comprise at least one amino acid substitution relative to the AP33 hypervariable loop sequence, although it is generally envisaged that the encoded polypeptide will have CDR or hypervariable loop sequences substantially identical, and preferably identical, to those in AP33.

Preferably the polynucleotide and/or the polypeptide of the invention will be isolated and/or purified. The term isolated is intended to indicate that the molecule is removed or separated from its normal or natural environment or has been produced in such a way that it is not present in its normal or natural environment. The term purified is intended to indicate that at least some contaminating molecules or substances have been removed. Preferably the polynucleotide and/or polypeptide are substantially purified, such that the relevant polynucleotide and/or polypeptide constitutes the dominant (i.e. most abundant) polynucleotide or polypeptide present in a composition.

The invention therefore preferably employs recombinant nucleic acids comprising an insert coding for a heavy chain variable domain and/or for a light chain variable domain of antibodies. By definition such nucleic acids comprise coding single stranded nucleic acids, double stranded nucleic acids consisting of said coding nucleic acids and of complementary nucleic acids thereto, or these complementary (single stranded) nucleic acids themselves.

Furthermore, nucleic acids encoding a heavy chain variable domain and/or for a light chain variable domain of antibodies can be enzymatically or chemically synthesised nucleic acids having the authentic sequence coding for a naturally-occurring heavy chain variable domain and/or for the light chain variable domain, or a mutant thereof. A mutant of the authentic sequence is a nucleic acid encoding a heavy chain variable domain and/or a light chain variable domain of the above-mentioned antibodies in which one or more amino acids are deleted or exchanged with one or more other amino acids. Preferably said modification(s) are outside the CDRs of the heavy chain variable domain and/or of the light chain variable domain of the antibody. Such a mutant nucleic acid is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly yeast, bacterial or mammalian cells, to obtain an optimal expression of the heavy chain variable domain and/or a light chain variable domain.

The invention further provides a nucleic acid construct comprising a polynucleotide in accordance with the first aspect of the invention. Typically the construct will be an expression vector allowing expression, in a suitable host, of the polypeptide(s) encoded by the polynucleotide. The construct may comprise, for example, one or more of the following: a promoter active in the host; one or more regulatory sequences, such as enhancers; an origin of replication; and a marker, preferably a selectable marker. The host may be a eukaryotic or prokaryotic host, although eukaryotic (and especially mammalian) hosts may be preferred. The selection of suitable promoters will obviously depend to some extent on the host cell used, but may include promoters from human viruses such as HSV, SV40, RSV and the like. Numerous promoters are known to those skilled in the art.

The construct may comprise a polynucleotide which encodes a polypeptide comprising three light chain hypervariable loops or three heavy chain hypervariable loops. Alternatively the polynucleotide may encode a polypeptide comprising three heavy chain hypervariable loops and three light chain hypervariable loops joined by a suitably flexible linker of appropriate length. Another possibility is that a single construct may comprise a polynucleotide encoding two separate polypeptides ― one comprising the light chain loops and one comprising the heavy chain loops. The separate polypeptides may be independently expressed or may form part of a single common operon.

Nucleic acid constructs encoding the HCV E2-binding polypeptides of the invention may be produced using standard recombinant nucleic acid techniques well known to those skilled in the art including, for example, oligonucleotide-directed site-directed mutagenesis (see, e.g. Carter et al, 1986 Proc. Natl. Acad. Sci. USA 83, 8127-8131) and PCR (Ho et al, Gene 1989 77,51-59).

The invention further provides a host cell, *in vitro,* comprising the polynucleotide or construct defined above. The host cell may be a bacterium, a yeast or other fungal cell, insect cell, a plant cell, or a mammalian cell. The invention may also provide a transgenic multicellular host organism which has been genetically manipulated so as to produce a polypeptide in accordance with the invention. The organism may be, for example, a transgenic mammalian organism (e.g. a transgenic goat or mouse line) or a transgenic plant line. Methods of producing the polypeptide are described further below.

### D. IMMUNOGENS

In a further aspect the invention provides a composition for inducing antibodies which bind to Hepatitis C Virus (HCV) E2 glycoprotein, the composition comprising:
a peptide having the amino acid residue sequence XLXNXXGXWXX; and a physiologically acceptable carrier, excipient or diluent;
the peptide optionally comprising additional amino acid residues at the N and/or C terminal but wherein the peptide does not encompass the entire HCV E2 glycoprotein nor the E2₆₆₀ fragment thereof (i.e. residues 384-660 of the HCV polyprotein), and wherein one or more of the amino acid residues may be covalently modified.

Two or more of the X residues in the sequence may be the same, or every X residue may be different.

X may be any of the naturally occurring amino acid residues or, less preferably, may be an unconventional residue (e.g. ornithine, citrulline, hydroxyproline, γ-Carboxyglutamate, O-Phosphoserine). Other covalent modifications which are envisaged include, in particular, glycosylation (especially N-glycosylation at one or more N residues).

For present purposes, the first residue X (i.e. that nearest the amino terminal) may be referred to as X₁, the second X residue as X₂, the third residue as X₃, and so on:
In a preferred embodiment, X₁ is S, E, Q, H, P or L.
In a preferred embodiment X₂ is V, I, A, R or F.
In a preferred embodiment X₃ is S, T, H, L or A.
In a preferred embodiment X₄ is N, Q or G.
In a preferred embodiment X₅ is S, K or T.
In a preferred embodiment X₆ is H, R or Q.
In a preferred embodiment X₇ is I, L, F or P.

Accordingly, examples of preferred amino acid sequences include the following:

| X₁ | X₂ | X₃ | X₄ | | X₅ | | X₆ | X₇ | | |
|---|---|---|---|---|---|---|---|---|---|---|
| S | | | | | | | | | | |
| E | V | S | | | | | | | | |
| Q | I | T | N | | S | | H | I | | |
| H | L | A | N | H | Q | G | K | W | R | L |
| P | | F | | A | G | | T | | Q | F |
| L | | R | | L | | | | | | P |

Particularly preferred sequences include the following: QLINTNGSWHI; QLVNTNGSWHI; QLINSNGSWHI; SLINTNGSWHI; ELINTNGSWHI; HLANHQGKWRL; PLFNANGTWQF; and ELRNLGGTWRP

In one embodiment the peptide substantially or essentially consists of the amino acid residue sequence XLXNXXGXWXX.

More preferably the peptide comprises an amino acid sequence conforming to the formula:
X₁LX₂NX₃NGSWHI or
X₁LX₂NX₃NGSWHIN

This sequence conforms to the conserved sequence of amino acid residue numbers 412-423 of the HCV E2 protein, in which typically only positions 412, 414 and 416 exhibit natural variation. Preferably X₁ is Q,S or E. Preferably X₂ is I or V. Preferably X₃ is T or S.

In one embodiment the peptide substantially or essentially consists of the amino acid residue sequence:
X₁LX₂NX₃NGSWHIN

In another, preferred, embodiment the peptide comprises additional amino acid residues. In particular, the peptide may contain one or more additional B or T cell peptide epitopes. In particular the peptide may contain one or more additional T helper cell peptide epitopes.

The additional amino acid residues may, for example, include one or more repeats of the sequence XLXNXXGXWXX and/or may contain peptide epitopes from other portions of the HCV E2 glycoprotein, and/or peptide epitopes from other HCV proteins or from other proteins entirely. For example, the peptide may be presented as part of a molecule, in which the amino acid residue sequence XLXNXXGXWXX is covalently coupled (typically, but not necessarily, by a peptide bond) to any other desirable moiety, such as a peptide or polypeptide containing one or more B or T cell epitopes. Conveniently such a molecule may be a fusion protein, which may be expressed and synthesised in a biological system (e.g. by a micro-organism or by a tissue culture system). The repeats of the peptide sequence may optionally be separated by an intervening spacer, or may be directly adjacent.

Alternatively the epitope sequence may be presented as a branched molecule comprising a plurality of repeats of the epitope sequence. In one embodiment the molecule comprises a bifurcating core of lysine and a C-terminal alanine residue to which are linked a plurality of copies of the epitope.

Where the epitope is present a plurality of times in the composition, the possibility arises of including variant sequences e.g. one peptide sequence may be such that X₁ is S and X₂ is V and another peptide sequence present in the molecule may be such that X₁ is Q and X₂ is I.

In a further aspect, the invention provides a nucleic acid construct which encodes a peptide having the amino acid residue sequence XLXNXXGXWXX, the peptide optionally comprising additional amino acid residues at the N and/or C terminal, but wherein the nucleic acid construct does not encode the entire HCV E2 glycoprotein or the E2₆₆₀ fragment thereof The construct will typically be an expression construct, comprising a promoter, such that the encoded peptide can be expressed in a suitable prokaryotic or eukaryotic host. Conveniently the promoter will be one which is operable in a mammalian, especially a human, host. Numerous suitable promoters are known to those skilled in the art (for example, cauliflower mosaic virus (CaMV) promoter, Rous sarcoma virus (RSV) promoter, dihydrofolate reductase (DHFR) promoter, retinoic acid receptor-β (RAR-β) promoter, human cytomegalovirus immediate-early gene-1 (HCMV) promoter, SV-40 promoter, human c-fos promoter).

In one particular embodiment, the nucleic acid construct encodes a peptide in which the amino acid sequence XLXNXXGXWXX is present a plurality of times, either as adjacent repeats or as repeats separated by an intervening spacer.

The construct may comprise one or more regulatory features, such as an enhancer, an origin of replication, and one or more markers (selectable or otherwise). The construct may take the form of a plasmid, a yeast artificial chromosome, a yeast mini-chromosome, or be integrated into all or part of the genome of a virus, especially an attenuated virus or similar which is non-pathogenic for humans.

The composition or the construct are conveniently formulated for safe administration to a mammalian, preferably human, subject. Typically, they will be provided in a plurality of aliquots, each aliquot containing sufficient composition or construct for effective immunisation of at least one normal adult human subject. If desired, a composition may be prepared which is in accordance with both the fourth and fifth aspects of the invention defined above.

The composition or construct may be provided in liquid or solid form, preferably as a freeze-dried powder which, typically, is rehydrated with a sterile aqueous liquid prior to use.

Preferably the composition or the construct will be formulated with an adjuvant or other component which has the effect of increasing the immune response of the subject (e.g. as measured by specific antibody titre) in response to administration of the composition or construct.

An adjuvant is a substance which causes antigen non-specific stimulation of the immune response. Known adjuvants include ADP-ribosylating bacterial toxins such as cholera toxin (CT) and *E*. *coli* heat labile toxin (LT), the non-toxic B sub-units thereof and toxoids (i.e. mutant molecules in which one or more mutations renders them non-toxic or molecules rendered non-toxic by chemical treatment, such as cross-linking of the A and B sub-units). Another known adjuvant is alum, which is approved for use in human vaccines. Other substances which may enhance the immune response include lipids, especially lipid-containing vesicles, liposomes, micelles and the like.

Administration of the construct to a subject results in the peptide epitope being expressed in at least some of the cells of the subject, which in turn can induce an immune response against the epitope (i.e. induce the development and/or expansion of a population of B cells which produce antibody which binds to the epitope). Such constructs are referred to generically as "DNA vaccines". The nucleic acid may be administered to the host by any appropriate route: e.g. intravenously, subcutaneously, or via needless administration into or through the skin. The nucleic acid may be administered in "naked" form or may be co-administered with other molecules or various types of particles, the nucleic acid being encapsulated within or associated in some way with the particles (e.g. bound to the surface, typically by electrostatic attraction). Particles which might be used include liposomes, virus particles, gold microparticles and the like. One virus particle which has been extensively used for research purposes in this general area is the Modified Vaccinia Ankara virus (MVA), which may be used as a vector to deliver the nucleic acid to host cells in the subject. MVA has so far been found to be safe. Other viruses which have been used as vectors include adenoviruses, and adeno-associated virus (AAV).

Those skilled in the art will appreciate that the construct need not be administered as DNA, but could in fact be administered as RNA as part of an RNA virus, which is then transcribed into DNA in a host cell and subsequently translated.

The composition may be administered to a subject by any suitable route. Oral, nasal or other mucosal routes are non-invasive and so may be preferred if they are found to be effective, but more conventional routes such as intravenous, subcutaneous or intramuscular injection are likely to elicit a stronger immune response. The optimum dose of the peptide to be administered may depend on the size and age of the subject, the route of administration etc. As a general guide, an effective dose (i.e. one which induces a detectable antibody titre in a subject who previously had no detectable antibody against the epitope; or which causes a detectable increase in antibody titre in a subject with some pre-existing antibody titre) will comprise an amount of the epitope in the range 50µg-500mg for an adult human, preferably in the range 100µg-250mg.

In one particular embodiment of the invention, there is provided a composition which is in accordance with the fourth aspect of the invention and which further comprises a nucleic acid construct in accordance with the fifth aspect of the invention.

The physiologically acceptable carrier, excipient or diluent may be solid or liquid. Suitable liquids include water and aqueous solutions, such as saline solution, phosphate-buffered saline and the like. Suitable solids include starches, dextrans and gels (e.g. carrageenans, alginates etc).

The composition and/or construct of the invention may be used to induce antibodies in a subject, which antibodies bind to HCV and which neutralise (i.e. render non-infective) the virus. For present purposes, an antibody can be considered neutralising if it can cause at least a 50% reduction in infectious titre of HCV in an *in vitro* assay when the antibody is pre-incubated with the virus at 37°C for 1 hour and the antibody has a concentration of not more than 100µg/ml, preferably not more than 75µg/ml. Accordingly, the composition and/or construct can be used to generate antibodies which may prevent infection, or provide limited protection by at least lessening the severity of infection, should the subject subsequently encounter HCV. Thus the composition/construct can be used to prevent disease entirely or at least ameliorate the symptoms of infection.

Alternatively, the composition/construct may be used among those already infected with HCV so as to enhance the immune response to HCV i.e. to treat disease. Such treatment may facilitate clearance of the virus from those subjects who are cutely or chronically infected.

Thus, in a further aspect the invention provides a method of preventing and/or treating HCV infection in a mammalian, preferably human, subject the method comprising administering an effective amount of a composition and/or a construct in accordance with the invention, so as to elicit or enhance the synthesis of HCV-neutralising antibody in the subject. The dose and route of administration may conveniently be as described previously.

As explained elsewhere, the inventors have surprisingly found that antibodies which bind to the epitope XLXNXXGXWXX are able to neutralise viruses representative of each known genotype of HCV. Accordingly, if such antibodies are present in a subject at sufficiently high concentration they should be able to protect against infection and/or disease caused by any genotype of HCV. One way of achieving a sufficiently high concentration of antibody is active immunisation. An alternative approach is passive immunity, in which pre-existing antibodies are administered to a subject.

Thus in a further aspect the invention provides a method of preventing and/or treating HCV infection in a mammalian, preferably human, subject the method comprising administering to the subject an effective amount of one or more HCV neutralising antibodies which bind to the epitope XLXNXXGXWXX. Such antibodies may conveniently be polypeptides in accordance with the third aspect of the invention defined above, and in particular chimeric or, preferably, humanised antibodies or antibody fragments comprising CDRs identical or substantially identical to those of AP33.

The antibody/ies may be administered, for example, in the form of immune serum or may more preferably be a purified recombinant or monoclonal antibody. Methods of producing sera or monoclonal antibodies with the desired specificity are routine and well-known to those skilled in the art. The antibody/antibodies may be administered by any suitable route including, but not limited to, intravenous or intramuscular injection, intraperitoneally, or transdermally.

Preferably the administered antibody/antibodies are substantially purified (e.g. preferably at least 95% homogeneity, more preferably at least 97% homogeneity, and most preferably at least 98% homogeneity, as judged by SDS-PAGE). The antibody/antibodies may be conveniently mixed or combined with a pharmaceutically acceptable carrier, excipient or diluent, such as saline, phosphate buffered saline, Ringer's solution, dextrose solution etc. and may optionally include thickening agents, such as gelatin, starches, alginates, and derivatised celluloses.

The passive immunisation regime may conveniently comprise administration of a plurality of antibodies with different specificity against HCV antigens and/or administration of antibody in combination with other antiviral therapeutic compounds. Recently such passive immunisation techniques have been used safely to treat HIV infection (Armbruster et al, 2004 J. Antimicrob. Chemother. 54, 915-920; Stiegler & Katinger 2003 J. Antimicrob. Chemother. 51, 757-759).

The active or passive immunisation methods of the invention should allow for the protection or treatment of individuals against infection with viruses of any of genotypes 1-6 of HCV, except for very occasional mutant isolates (such as that exemplified by UKN5.14.4, below) which contain several amino acid differences to that of the consensus peptide epitope defined above.

In yet further aspects the invention provides respectively a diagnostic test apparatus and method for detecting the presence of HCV. The apparatus may comprise, as a reagent, one or more antibodies which bind to the epitope XLXNXXGXWXX. The antibody/ies may, for example, be immobilised on a solid support (e.g. on a microtitre assay plate, or on a particulate support) and serve to "capture" HCV particles from a sample (e.g. a blood or serum sample or other clinical specimen such as a liver biopsy). The captured virus particles could then be detected by, for example, adding a further, labelled, reagent which binds to the captured virus particles. Conveniently, the assay may take the form of an ELISA, especially a sandwich-type ELISA, but any other assay format could in principle be adopted (e.g. radioimmunoassay, Western blot) including immunochromatographic or dipstick-type assays.

The antibody/ies which bind to the XLXNXXGXWXX epitope may be labelled or unlabelled. Any suitable label may be employed e.g. radio-label, enzyme label, fluorescent label, or dye-loaded particulate label.

The assay method of the invention comprises the use of antibody which binds to the epitope XLXNXXGXWXX.

Since the antibodies can bind to HCV from any of genotypes 1-6, the assay apparatus and corresponding method should be capable of detecting in a sample HCV representative from any of these genotypes.

In a final aspect, it is possible that molecules comprising a peptide conforming to the general formula XLXNXXGXWXX may be able to inhibit HCV entry into susceptible cells by interfering with the fusion process. Thus, for example, peptides or polypeptides containing the aforementioned amino acid residue sequence may have clinical usefulness, and the invention thus encompasses pharmaceutical compositions comprising such molecules in admixture with a physiologically acceptable diluent, excipient or carrier. Such an approach has been described recently for T20 peptide, which corresponds to a portion of HIV gp41 (Zwick et al, 2004 Nature Medicine 10, 133-134).

For the avoidance of doubt, it is hereby expressly stated that the invention may comprise any feature described herein as "preferred", "advantageous", "convenient" or the like in isolation, or in combination with any other feature or features so described, unless the context dictates otherwise. Further, the content of all publications mentioned in this specification is incorporated herein by reference.

The invention will now be described further by way of illustrative example.

### Examples

### Example 1

The isolation of cDNA sequences encoding E1E2 glycoproteins from patients infected with different genotypes of HCV was reported by Lavillette *et al.,* (43). To generate HCV pseudoparticles (HCVpps) enveloped with glycoproteins derived from different genotypes, the inventors used vectors expressing appropriate HCV E1E2 and murine leukaemia virus (MLV) Gag-Pol. The inventors also utilised a MLV transfer vector encoding the GFP reporter protein to act as a transduction marker.

The plasmids expressing the HCV genotype 1a strain H-derived full-length E1E2, murine leukaemia virus (MLV) Gag-Pol, and the MLV transfer vector carrying GFP under the control of human CMV promoter have been described previously (6). The cDNA sequences encoding the full-length E1E2 of HCV from various clinical isolates [representing amino acid residues 170 to 746 of the HCV open reading frame referenced to strain H77c (70)] were generated by PCR, cloned downstream from a human CMV promoter in the expression vector pCR3.1 (Invitrogen) or phCMV-7a (6), and their nucleotide sequence determined as described (43).

Experiments involved the use of human epithelial kidney (HEK) 293T cells (ATCC CRL-1573) and human hepatoma (Hub-7) cells. These were grown in Dulbecco's modified Eagle's medium (DMEM, GIBCO BRL) supplemented with 10% foetal calf serum (FCS), 5% non-essential amino acids, glutamine and penicillin/streptomycin.

HCVpps were produced essentially as described previously (6). Briefly, HEK293T cells were co-transfected with the MLV Gag-Pol packaging vector, the MLV-GFP transfer construct, and a plasmid expressing HCV E1E2, using the calcium phosphate transfection method (Sigma). In all experiments a no-envelope control was used in which the HCV glycoprotein-expressing construct was excluded from the co-transfections of HEK293T cells. Two days following transfection, the medium containing HCVpps was collected, clarified, filtered through 0.45 *µ*m pore-sized membrane and used for infection of Huh-7 cells. Four days following infection, the cells were harvested and analysed on a FACSCalibur (Becton Dickinson) using CellQuest software. The transduction efficiency was determined as the percentage of GFP-positive cells (following subtraction of the number of GFP-positive Huh-7 cells 'infected' with the no-envelope control which was typically 0.05%). The infectious titres, expressed as transducing units per ml, were calculated from the transduction efficiency.

The E1E2 sequences from the established infectious clone type 1a strain H (6) were used as control alongside the patient-derived E1E2 clones throughout this series of experiments. A total of 289 patient isolates were screened and, of these, 39 were able to render pseudoparticles infectious. A representative selection of these functional clones, the properties of some of which were reported recently by the inventors (43), are shown in Table 1. At least one infectious clone representative of each of the genotypes was identified. HCVpps derived from some patient isolates were reproducibly more infectious in this assay than those from the control genotype 1a strain H. For example, HCVpps derived from the construct UKN2B1.1 regularly transduced over 30% of the target cells, whereas those derived from H77 only transduced 10-20%. Typically, the infectious titres, expressed as transducing units per millilitre (TU/ml), were about 1 × 10⁵ TU/ml for UKN2B1.1 and 4 × 10⁴ TU/ml for type 1a strain H. In contrast, the genotype 3, 5 and 6 HCVpps gave very low titres (1 to 4 x 10³TU/ml) (Table 1).

**Table 1. Transduction efficiency of HCVpp carrying E1E2 of diverse genotypes**

| **Genotype** | **Strain or Construct** | **% GFP positive** | **TU/ml** |
|---|---|---|---|
| | | **cells*** | X10⁴ |
| 1a | Strain H | 12.5 | 4.3 |
| 1a | UKN1A14.8 | 12.5 | 4.3 |
| 1a | UKN1A14.36 | 21.5 | 7.2 |
| 1b | UKN1B12.6 | 26.2 | 8.7 |
| 2a | UKN2A2.4 | 20.0 | 6.6 |
| 2b | UKN2B1.1 | 32.5 | 10.8 |
| 2b | UKN2B2.8 | 11.6 | 3.8 |
| 3a | UKN3A13.6 | 0.3 | 0.11 |
| 4 | UKN4.11.1 | 31.2 | 10.3 |
| 4 | UKN4.21.16 | 31.6 | 10.5 |
| 4 | UKN4.21.17 | 24.8 | 8.2 |
| 5 | UKN5.14.4 | 0.35 | 0.11 |
| 5 | UKN5.15.11 | 0.7 | 0.24 |
| 6 | UKN6.5.340 | 1.14 | 0.4 |

| | | | |
|---|---|---|---|
| *The transduction efficiency was calculated after subtracting the number of GFP-positive cells resulting from 'infection' with no-envelope control. These are average values derived from 2 or more independent experiments. | | | |

### Example 2

To investigate why many of the isolates lacked infectivity, the inventors checked whether HCV glycoproteins were expressed in the transfected HEK293T cells. The relative level of the E2 glycoprotein in each cell lysate was determined by means of an ELISA involving GNA (*Galanthus nivalis*) lectin-coated ELISA plates (Dynex Labsystems), and polyclonal rabbit serum R646, and two monoclonal antibodies (MAbs), AP33 and ALP98, all raised against type 1a E2 and described previously 52, 16). MAb AP33 and R646 antiserum were purified on a protein G column according to the manufacturer's protocol (Amersham Biosciences).

The ELISA assay to detect E2 glycoprotein was performed essentially as described previously (54). Briefly, the E1E2 glycoproteins from the clarified lysates of HEK293T cells co-transfected as described above were serially diluted threefold and captured on to GNA lectin-coated ELISA plates. The bound glycoproteins were detected using anti-E2 MAbs AP33 or ALP98 or rabbit polyclonal serum R646, followed by an anti-species IgG-HRP (Sigma) and TMB (3, 3', 5, 5' ― Tetramethyl-Benzidine, Sigma) substrate. Absorbance values were determined at 450 nm.

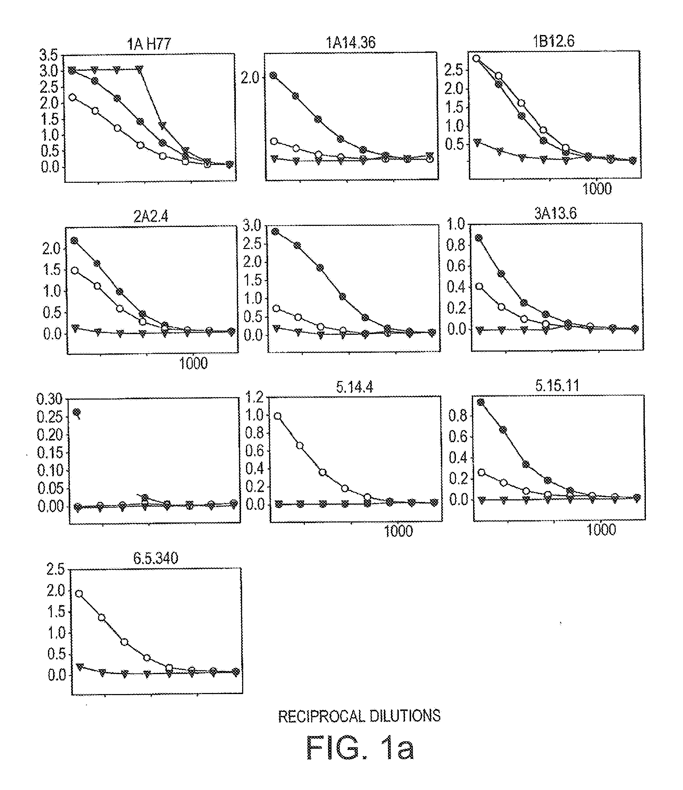

The results are shown in Figure 1a, which is a series of graphs of absorbance (arbitrary units) against reciprocal of dilution, for the various lysates tested. Results for detection with AP33 are denoted by filled circles, ALP98 by empty circles, and those for R646 polyclonal antiserum by filled triangles.

Lysates of HEK293T cells co-transfected with each of the infectious clones together with the MLV Gag-Pol and the GFP reporter constructs contained levels of E2 that gave a strong, concentration-dependent signal with at least one of the two MAbs. It is noteworthy that type 4 isolate (UKN4.21.16), and one of the two type 5 isolates tested (UKN5.14.4) were not recognised by the MAbs ALP98 and AP33, respectively. This is due to the presence of variant amino acids within the epitopes recognised by these antibodies (see below). The rabbit antiserum R646 almost exclusively recognised the genotype 1a strain H E2; it failed to recognise not only E2 from other genotypes, but also that from a different isolate of the same subtype (1A.4.36).

Lysates of HEK cells transfected with all the isolates that did not yield infectious HCVpps were also analysed by GNA ELISA for the presence of E2. While some had no detectable or low levels of E2, others contained levels of E2 similar to those found in lysates of cells transfected with infectious clones (data not shown). The inventors concluded that some isolates lack infectivity because they do not express E2, whereas others are non-infectious despite expressing high levels of E2, presumably because the E2 or the E1E2 complex that they encode is non-functional in some way.

### Example 3

The HCVpp infectivity is dependent on the incorporation of the full-length E1E2 complex into the envelope of the particles (6, 39). Although the ELISA data above confirmed the presence of E2 derived from different genotypes, presence of E1 could not be analysed due to the lack of a broadly reactive anti-E1 antibody. Instead, the inventors investigated E1E2 complex formation by immunoprecipitation assay. HEK293T cells co-transfected with the HCV glycoprotein-expressing constructs and the MLV Gag-Pol and GFP transfer vector were radiolabelled with [³⁵S]methionine/cysteine.

Radiolabelling was performed as follows.

Eighteen hours following transfection, cells were washed with PBS, and incubated in methionine/cysteine-free medium containing 25 µCi/ml of L-[³⁵S] Redivue™ Pro-Mix™ (Amersham Biosciences) for 48 h. The medium of transfected cells was harvested and clarified by centrifugation. The cells were washed with PBS, lysed in lysis buffer (20 mM Tris-HCl, pH 7.4, 20 mM iodoacetamide, 150 mM NaCl, 1 mM EDTA, 0.5% Triton X-100), and the lysate spun briefly to remove nuclei. The clarified cell lysates and the medium containing HCVpps were incubated with a mixture anti-E2 MAbs AP33 and ALP98 for 2 h at 4°C and the resulting immune complexes precipitated using protein A-sepharose. Following washes of the protein A-sepharose beads, the immune complexes were released into SDS-PAGE denaturation buffer (200 mM Tris-HCl, pH6.7; 0.5% SDS; 10% glycerol; 20 mM DTT) and analysed by SDS-10% PAGE. The gels were dried and exposed overnight to a phosphor screen and the radiolabelled proteins visualised with a Bio-Rad Personal FX phosphorimager.

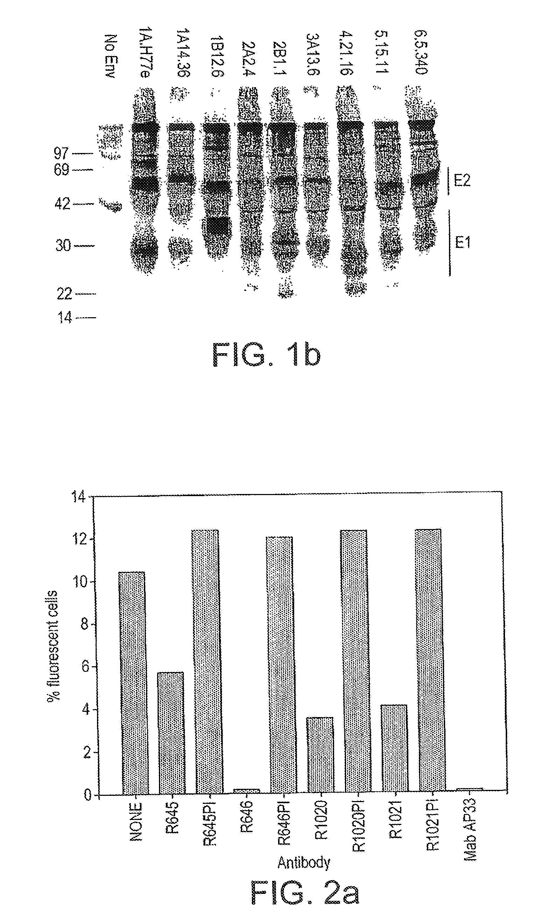

The results are shown in Figure 1b.

E1 was co-immunoprecipitated along with E2 from the lysates of cells transfected with most of the glycoprotein-expressing constructs. There was a degree of variation in the relative amounts of the proteins produced and interesting differences in the molecular weight of the precipitated proteins (particularly E1) were apparent. These are most likely due to differential glycosylation, as nucleotide sequence analysis show variations in the predicted glycosylation sites between different genotypes (43). It is noteworthy that there was a significant variation in the relative stoichiometry between E1 and E2 of different genotypes. Similarly, E1E2 complexes secreted into the medium (a proportion of which were expected to be in the form of HCVpps) of the transfected cells were also detected by immunoprecipitation with the same MAbs (data not shown).

### Example 4

*Antibody-mediated neutralisation of HCVₚₚ infection of target cells:* The inventors tested the ability of MAb AP33, rabbit antisera R645 and R646 (both raised against the soluble ectodomain of type 1a strain H77c) to inhibit strain H77c HCVpp infection of cells.

Also tested were the antisera R1020 and R1021 raised in New Zealand rabbits immunised with a branched peptide corresponding to the hypervariable HVR-1 region (residues 384-411) of the genotype 1a strain H77c. The immunisation protocol used to generate these antisera has been described previously (53).

The neutralisation assay was performed as follows. HCVpps harbouring the genotype 1a strain H E1E2 were pre-incubated for 1 hour at 37°C with 1:120 dilutions of anti-E2 sera R645, R646, R1020, and R1021 or their pre-immune (PI) counterparts, or 50 *µ*g/ml MAb AP33. The virus/antibody mixture was then added to Huh-7 cells plated in a 6-well tissue culture dish and the cells incubated at 37°C for 3 h. Following removal of the inoculum, the cells were re-fed with fresh medium and incubated at 37°C for 4 days. The proportion of infected cells was determined by measurement of GFP by FACS as described above. The neutralising activity was expressed as IC₅₀ or IC₉₀, defined as the concentration of antibody required to achieve 50% or 90% inhibition, respectively, of infection.

The results are shown in Figure 2A.

Of the rabbit antisera tested, R646 was able to completely abrogate infection whereas R645 blocked infection to approximately 50%. Both R1020 and R1021 anti-HVR1 antisera were able to neutralise infection by 65%. As expected, the corresponding pre-immune rabbit sera had no effect on HCVpp infection. Similar to R646, the MAb AP33 completely blocked infection of Huh-7 cells by strain H77c HCVpp.

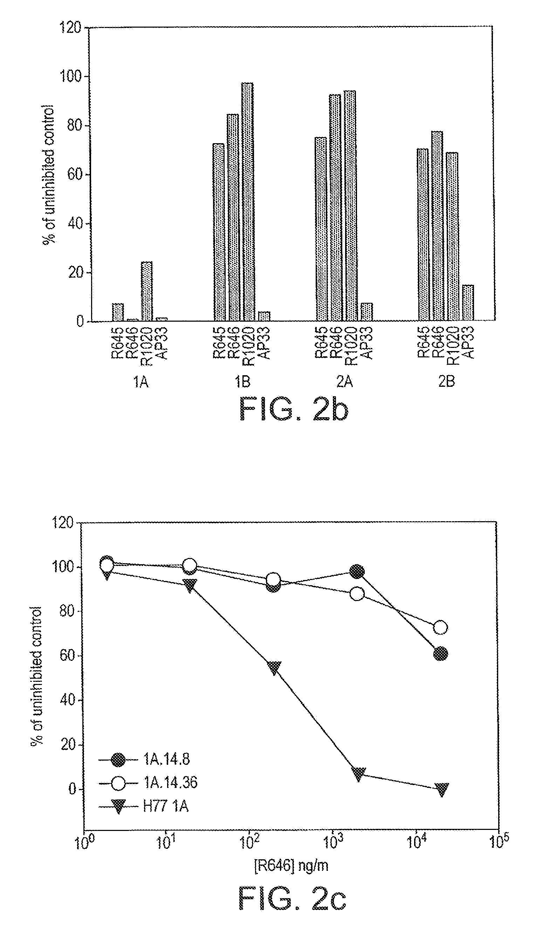

The inventors next tested the ability of these antisera and the MAb AP33 to neutralise HCV genotypes other than 1a strain H77. They found that AP33 was broadly cross-neutralising, whereas antisera R645, R646 and R1020 had very little effect on the infectivity of HCVpps incorporating the type 1b, 2a or 2b glycoproteins (Fig. 2b). Having established that R646 does not significantly inhibit infection by HCVpp derived from genotypes other than 1a, the inventors checked whether its specificity was limited to glycoproteins of type 1a strain H, or whether it could inhibit other type 1a isolates. As shown in Fig. 2c, this antiserum was less effective on the two patient-derived isolates 1A14.8 and 1A14.36 than on 1a strain H, against which it had an IC₅₀ value of 200 ng IgG/ml.

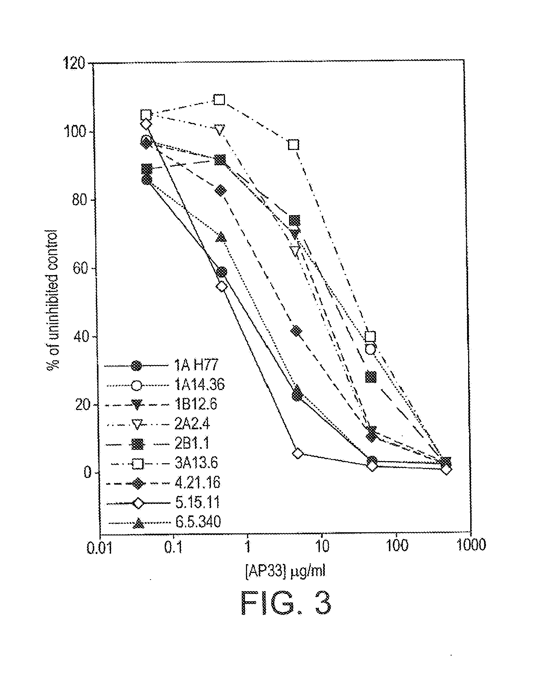

The broad reactivity of MAb AP33 was examined further by testing it over a range of concentrations on a panel of HCVpps incorporating E1E2 derived from isolates representing the full complement of genotypes. As shown in Fig. 3, HCVpps harbouring MAb AP33-reactive E2 of isolates representing all 6 genotypes were effectively neutralised, with the IC₅₀ ranging from 0.6 µg/ml for type 5 to 22 µg/ml for genotype 3a. The infectivity of one of the genotype 5 HCVpp isolates, UKN5.14.4, was not affected by MAb AP33 (data not shown) since the E2 glycoprotein of this infectious isolate is not recognised by this antibody (Figure 1A).

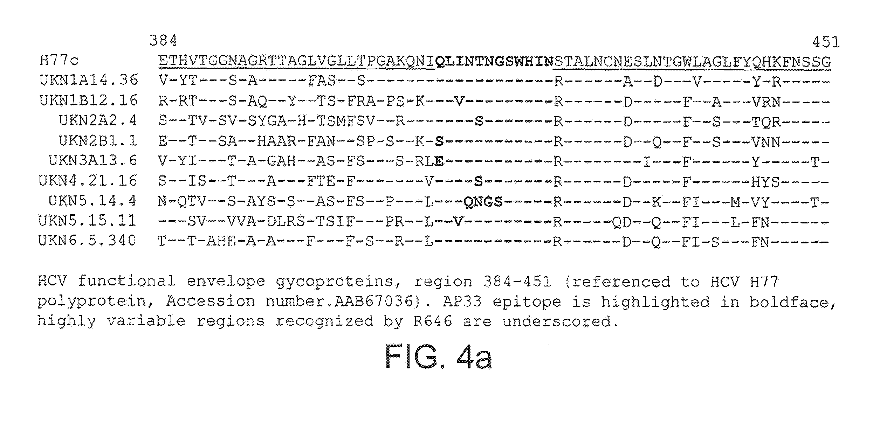

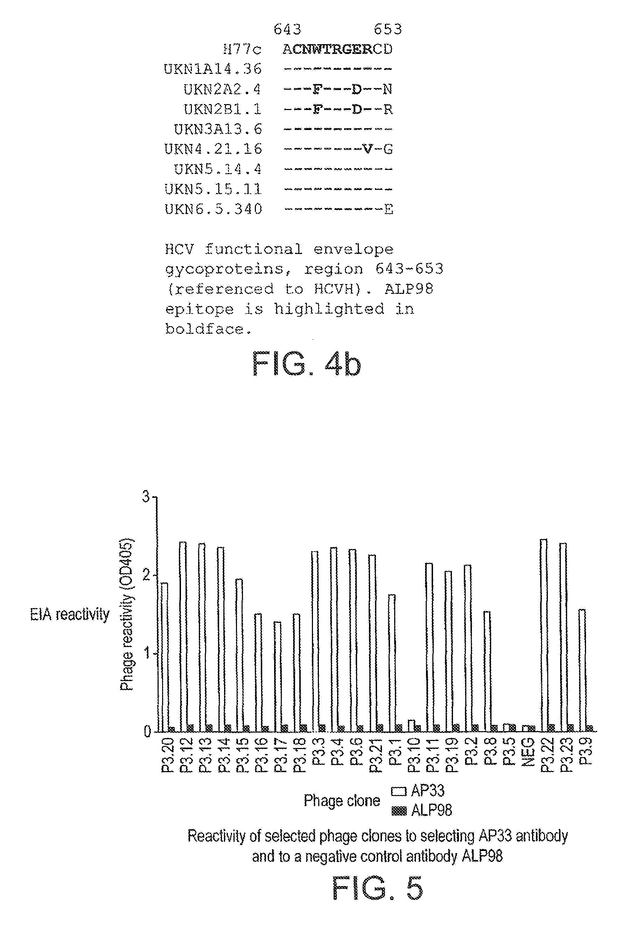

Amino acid sequence alignment (Figure 4a) of the N-terminal region of E2 of the different genotypes used in this study showed that the MAb AP33 epitope (residues 412 ― 423) is relatively well conserved with only three variant amino acid sites (residues 412, 414 and 416). Other sites within this epitope are absolutely conserved. An exception to this was the corresponding amino acid sequence in the E2 encoded by the genotype 5 isolate UKN5.1.4.4; it has a 4 residue change in the sequence with a -1 shift in the potential glycosylation site (NGS) relative to that of the other isolates (Fig. 4a). In contrast, significant variations were seen between different genotypes in sequences corresponding to the genotype 1a strain H linear epitopes recognised by R646 consistent with its restricted specificity. Finally, the epitope recognised by the ALP98 antibody (residues 644 ― 651) was also conserved although an arginine to valine mutation present at position 651 in the genotype 4 clones abolished recognition by this antibody (Fig. 4b). To investigate the epitope recognised by AP33 more closely, the epitope was fmely mapped.

### Example 5 Fine mapping of AP33 contact residues using a phage biopanning screen:

The PhD (New England Biolabs [NEB]) series of phage displayed random peptide libraries (RPDLs) are based in the type 3 phage peptide display vector M13KE. Individual phage within these libraries express up to five copies of a random peptide fused to the N-terminus of the mature pIII capsid protein via the spacer sequence Gly-Gly-Gly-Ser. The library expressing random 12mer peptides was used in our experiments (NEB catalogue No. E8110S). Affinity selection of the PhD phage-library was performed essentially as described by the manufacturer. In brief, AP33 was coated at a concentration of 10 - 100 µgml⁻¹ by overnight incubation in 100 µl of coating buffer (CB) (0.05 M carbonate-bicarbonate, pH 9.6) at 4°C onto wells of a maxisorp microtitre plate (Nunc, Roskilde, Denmark). Following coating, the antibody solution was discarded and the wells were then blocked for 1 hour at 4°C with 300 µl of TBS-TB (Tris-buffered saline, pH 7.6 [TBS] containing 0.1% (v/v) Tween 20 and 5% (w/v) milk powder (Marvel^{RTM}, Cadbury's). After discarding the blocking solution, wells were washed six times with TBS-T (TBS containing 0.1% (v/v) Tween 20) before the addition of 1 × 10¹¹ phage from the PhD library diluted in 100 µl TBS-T. These phage were allowed to bind immobilised AP33 for 1 hour at 25°C before the unbound phage were removed through serial washes with TBS-T. Bound phage were eluted with 100 µl of 0.2M Glycine-HCl (pH2.2) for 10 minutes, transferred to a microfuge tube, then neutralised by addition of 15µl of 1M Tris-HCl (pH9.1).

Recovered phage were titrated by preparing serial dilutions of eluted phage in LB and 10.0 µl of each dilution added to 250 µl of E. *coli* strain ER2537 (NEB) during the logarithmic phase of growth (0.3 OD₆₀₀ₙₘ). Phage were allowed to infect the bacteria for 5 minutes at 25°C before bacteria were mixed with 3 ml of 0.7 % agar, containing 1 mM X-Gal and 1 mM IPTG and overlaid onto LB agar plates. The agar was allowed to solidify for 5 minutes before inverting the plates and incubating at 37°C overnight. Titres of phage were subsequently calculated from the mean number of blue plaques formed using equation: Phage µl⁻¹ = ([mean number of blue plaques] x [dilution]⁻¹) / 10.

The enriched phage library was then expanded by adding 50 µl of the eluted phage (approximately 1 x 10⁵ PFU) into 20 ml of log phase *E. coli* strain ER2537 in LB and incubating for at least 4.5 hours at 37°C. Following phage growth, bacteria and other debris were removed by centrifugation at 10 000 x g for 10 minutes (Sorvall SS-34). Phage were precipitated from the supernatant of this culture by the addition of 1/5^{th} volume of PEG (20 % [w/v] polyethylene glycol-8000, 2.5 M sodium chloride) for 1 hour at 4°C and collected by centrifugation at 15000 x g for 20 minutes. Phage pellets were then re-suspended in 1.0 ml TBS before a second precipitation with 1/5^{th} volume of PEG, for 1 hour on ice. Phage were subsequently collected by centrifugation at 10 000 x g for 20 minutes (MSE Micro Centaur) and re-suspended in a final volume of 200 µl sterile TBS. The concentration of phage within the expanded library was determined by titration as described above and 1 x 10¹² phage subsequently used as the input to a further 2 - 3 rounds of affinity selection.

Following the final round of affinity selection individual plaques obtained from titrations of eluted phage were inoculated into a 2 ml log-phase culture of ER2537 using a sterile pipette tip and grown at 37°C for 4.5 hours. Phage were recovered from the supernatant of this culture by two rounds of precipitation as described above and re-suspended in 100 µl TBS. Phage stocks were stored at -20°C.

To determine the antigenicity of the peptides enriched from the 12mer naive RPDL, an enzyme-linked immunoabsorbent assay (EIA) was used. AP33, diluted to between 1.0 - 100 µgml⁻¹ in 50 µl CB, was coated directly onto microtitre plate wells by overnight incubation at 4°C. Following blocking (as described above), approximately 1 × 10¹¹ phage particles were added to the wells and allowed to bind for 1 - 2 hours. Bound phage were then detected by incubation with anti-fd (Sigma, catalogue No. B-7786) diluted to 1:1000 in TBS-T. The binding of these antibodies was then detected by sequential incubations with an alkaline phosphatase conjugated anti-rabbit secondary antibody and pNPP substrate (Sigma) and the OD read at 490 nm.

Sequencing template DNA was prepared by PCR amplification of the DNA from approximately 1 x 10⁹ phage using primer gIII (f) 5'-ATTCCTTTAGTGGTACCTTTC-3' in conjunction with the -96 sequencing primer supplied by the manufacturer. A standard polymerase chain reaction (PCR) was used for amplification of DNA from both phage particles and bacteria. For each reaction, 2.5 µl of 10 x PCR buffer (100 mM Tris-HCl, 15 mM MgCl₂, 500 mM KCl [pH 8.3]), 5.0 µl (5.0 pmol) of each primer, 0.1 µl (0.5 units) of HotStar Taq polymerase (Qiagen) and 0.5 µl of a 10.0 mM dNTP mix (containing 2.5 mM dATP, dCTP, dGTP and dTTP) was added to a thin-walled microcentrifuge tube. The reaction volume was made up to 25.0 µl with DNAse/RNAse free water before the addition of 1.0 µl of phage particles diluted to approximately 1 × 10⁹ phage ml⁻¹ with DNA-ree water. DNA was then amplified by PCR cycling (35 cycles of 95°C, 45 seconds, 50°C, 45 seconds, 72°C, 90 seconds) on a PTC-100/200 thermal cycler (MJ Research).

Following amplification, unincorporated nucleotides and oligonucleotides were removed prior to sequencing by the addition of 1.0 µl (1.0 unit) each of shrimp alkaline phosphatase (SAP) and Exonuclease I (Exo I), followed by incubation at 37°C for 30 minutes. SAP and Exo I enzymes were then inactivated at 75°C for 15 minutes. 5.0 µl (approximately 100 - 500 ng) of template DNA from SAP/Exo I-treated PCR products were mixed with 4.0 µl of BigDye terminator ready reaction mix (Applied Biosystems) and 1.0 µl (1 pmol) of the -96 sequencing primer in a thin-walled PCR tube and cycle-sequencing (25 cycles of 96°C, 10 seconds, 50°C, 5 seconds, 60° C, 4 minutes) performed using a Perkin-Elmer 9600 thermal cycler. 10.0 µl DNAse free water and 2.0 µl (0.1 volumes) of 3 M sodium acetate (pH 5.2) were then added to the tube, before centrifuging briefly and transferring to a sterile microcentrifuge tube. The sequenced DNA was then precipitated by the addition 45.0 µl of 100 % ethanol and incubation for 10 minutes at 25°C. The precipitated DNA was recovered by centrifugation at 15 000 x g for 20 minutes and washed with 45.0 µl of 70 % ethanol before drying at 37°C for 15 minutes. Sequence analysis was subsequently performed using an ABI Prism 310 genetic analyser.

Identification of conserved residues of the EIA-reactive enriched displayed peptides that are likely to form contacts with the AP33 paratope was performed by computer-assisted alignment of the deduced peptides.

Following three rounds of selective biopanning against immobilised AP33, 22 phage clones were harvested, grown, and their reactivity to the AP33 antibody determined by EIA. (Figure 5). Figure 5 shows that the peptides displayed by the majority of enriched phage specifically interact with the AP33 antibody. Specificity of interaction is confirmed by the lack of reactivity to the control ALP98 antibody.

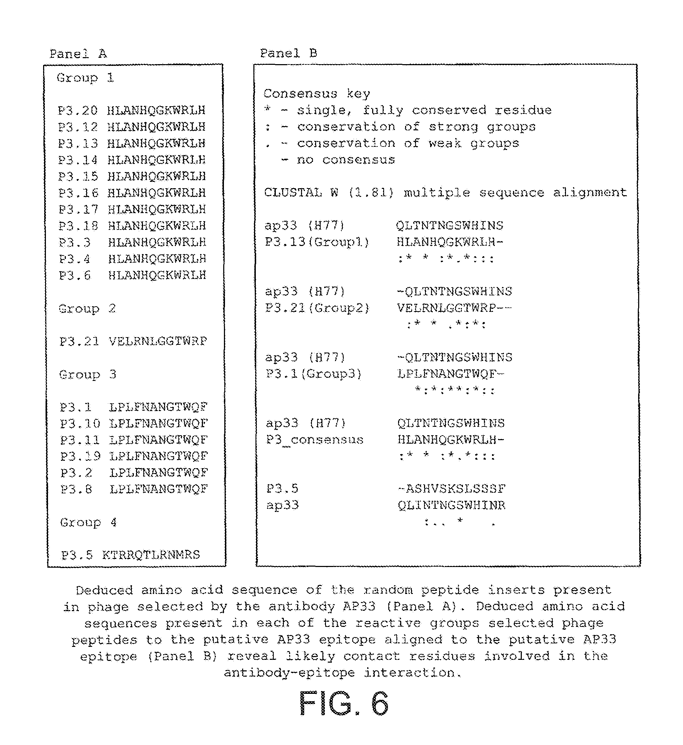

To identify residues most likely involved in mediating binding of the peptides to the AP33 peptide, DNA sequencing of the selected peptides was performed and their deduced amino acid sequences aligned using computer-assisted alignment. Three groups of phage were identified, each group containing a unique peptide sequence (Figure 6). Their consensus sequence, together with alignments of each group to the predicted AP33 epitope, indicate that the critical binding motif can be defined as XLXNXXGXWXX

In Figure 6 the deduced amino acid sequences of the random peptide inserts present in phage selected by the antibody AP33 is shown in Panel A. Deduced amino acid sequences present in each of the reactive groups selected phage peptides aligned (Panel B) to the putative AP33 epitope reveal the conserved contact residues involved in the antibody-epitope interaction. Note clone P3.5 was non-reactive in the AP33 EIA (Figure 5) and this peptide show minimal homology to the AP33 epitope.

### Example 6 ― Fine mapping of AP33 and 3/11 epitopes using alanine replacement mutant E1E2 proteins

The findings described above in Example 5 were further reinforced by experiments using a panel of H77 E1E2 mutant clones, in which one residue at every position in the putative AP33 epitope was substituted by alanine. This panel was also used to investigate the binding of a rat monoclonal antibody 3/11, which has been described as binding to the same epitope (Flint et al, 1999 J. Virol. 73, 6235-6244). Both 3/11 and AP33 were purified from hybridoma supernatants using a protein G column.

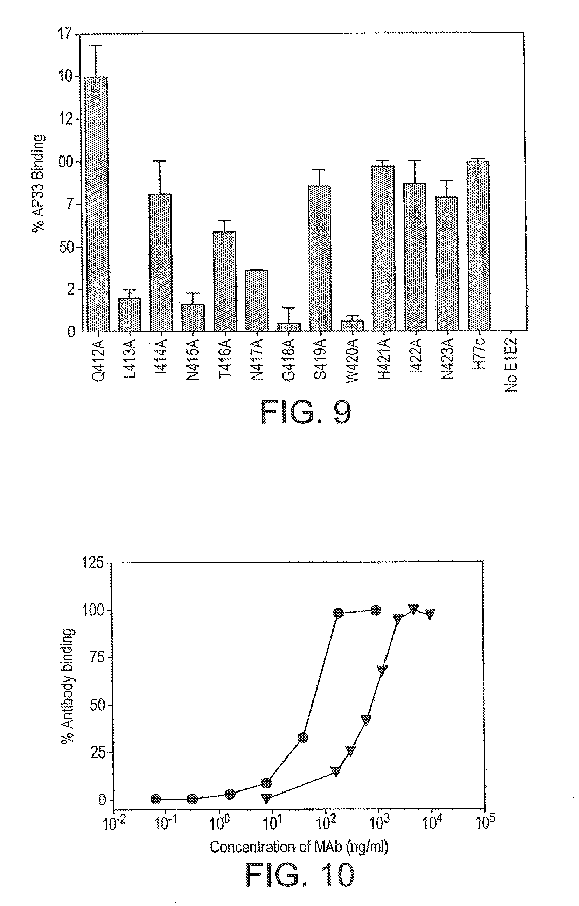

The various E1E2 mutants were expressed in HEK 293T cells and reactivity of the resulting proteins to MAb AP33 and 3/11 assessed using a GNA capture EIA (Figure 9). Binding of AP33 was reduced by more than 75% compared to wild type for mutants L413A, N415A, G418A and W420A, indicating that these residues were very important for binding. Mutation T416A and N417A also reduced AP33 binding, although this effect was not as marked as for the other four mutations. Substitution of glutamine by alanine at position 412 (Q412A) consistently enhanced binding of AP33 to E1E2 by approximately 50% compared to the wild type H77 protein. By contrast, this substitution had no effect on MAb 3/11 binding. Alanine replacement of the remaining five residues had negligible effect on AP33 recognition.

Consistent and significant reduction of binding by MAb 3/11 compared to wild type H77 protein was observed for mutant N415A, W420A and H421A, highlighting the importance of these residues in binding by MAb 3/11 (data omitted for brevity). Substitution of the isoleucine at position 422 resulting in moderate enhancement of 3/11 binding. Alanine replacement of the remaining residues either had no effect, or resulting in moderate reductions in binding, compared to wild type.

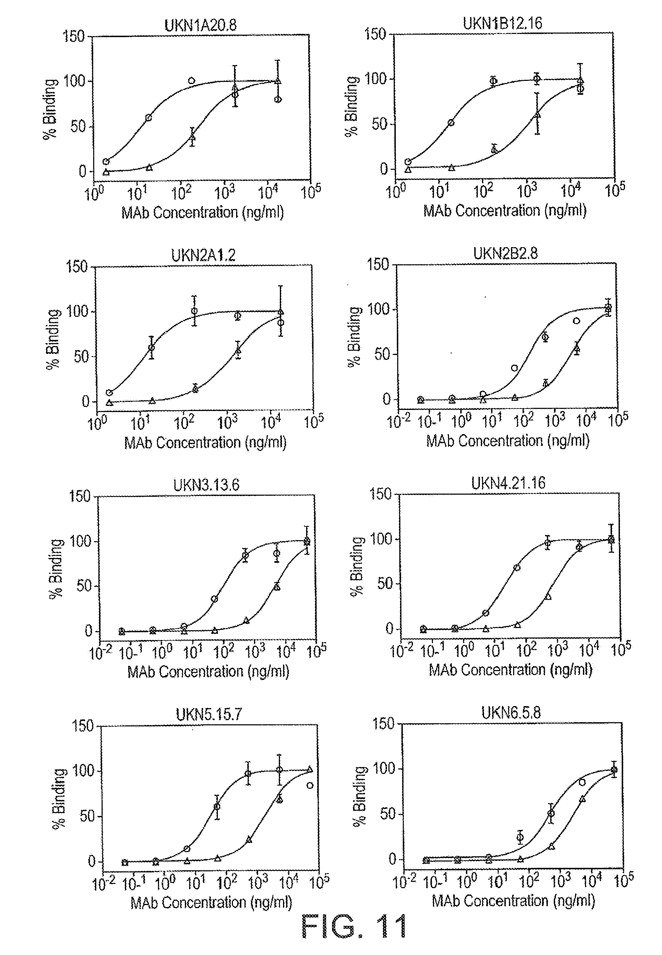

***Comparison of binding affinities and HCVₚₚ neutralisation efficiencies of MAb** AP33* ***and MAb 3*/*11:*** The fine epitope mapping experiments indicated that MAbs AP33 and 3/11 were recognising different contact residues within the E2 protein, so the inventors went on to assess whether or not these differences might translate into differences in binding affinity or neutralising potency. The concentration of MAb AP33 required to obtain 50% binding to a 412-423 branched peptide was more than 10-fold lower than that required for MAb 3/11 (Figure 10; AP33 = circles, 3/11 = triangles). Similarly, in biotinylated MAb binding assays, AP33 was more efficient at competing for binding than 3/11 (data not shown). Compared to MAb 3/11, competition with MAb AP33 resulted in greater reduction in binding by both biotinylated MAb AP33 and 3/11. The MAbs also exhibited marked differences in affinity to E1E2 representative of diverse HCV genotypes (Figure 11). Concentrations of MAb AP33 needed to obtain 50% binding to the E1E2 proteins ranged from approximately 1×10¹ to 1×10³ ng/ml, whereas 50% binding was achievable using 3/11 at concentrations ranging from approximately 1×10² to 1×10⁴ ng/ml. Together, these data indicate that AP33 has a higher affinity for E2 than MAb 3/11.

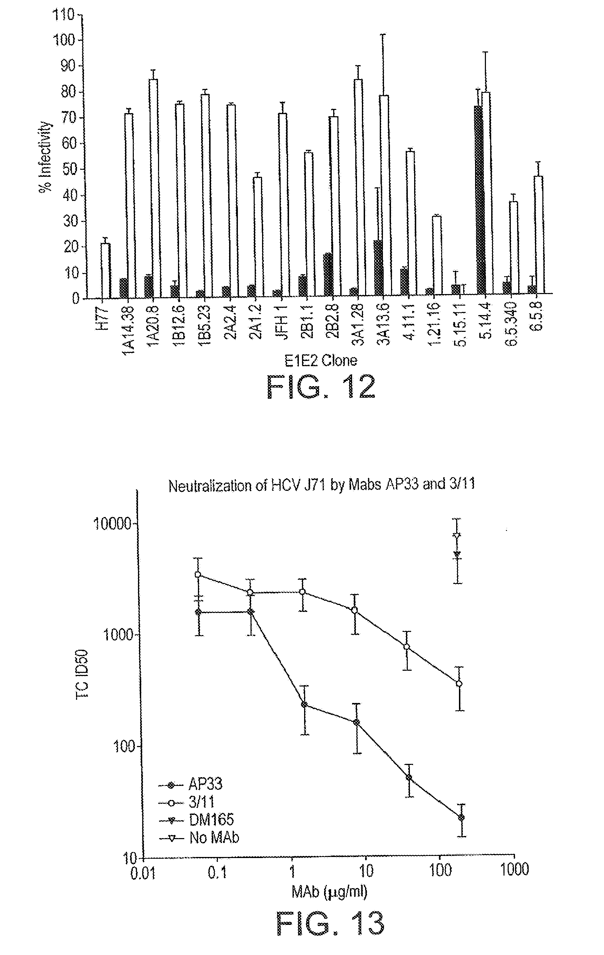

Similarly, a comparison of the ability of MAbs AP33 and 3/11 to neutralise HCVpp carrying E1E2 representative of genotypes 1-6 (Figure 12) showed that, whilst both antibodies were capable of broad neutralisation, neutralisation potency of AP33 was consistently greater than MAb 3/11 (p<0.001, Wilcoxon's matched pairs test). When used at a concentration of 50 µg/ml, MAb AP33 was able to neutralise HCVpp infectivity by between 80 and 99%. By contrast, the same concentration of MAb 3/11 only resulted in between 10-80% neutralisation. As described herein, MAb AP33 poorly neutralised HCVpp carrying E1E2 from the genotype 5 strain UKN.5.14.4; similarly MAb 3/11 was also unable to neutralise HCVpp carrying this E1E2 clone. This isolate has a 4 amino acid change (QLIQNGSSWHIN) in the E2 region corresponding to residues 412 ― 423. This mutation alters two of the residues important for AP33 (N415 and G418) recognition and one (N415) for 3/11. Both MAbs AP33 and 3/11 fail to react with UKN5.15.4 E2. Therefore, unsurprisingly, both MAbs also fail to neutralise UKN5.14.4 HCVpp.

### Example 7 ― Sequence analysis of the V_{H} and V_{L} regions of AP33 and 3/11

mRNA from approximately 10⁶ hybridoma cells was isolated using the RNeasy minikit (Qiagen), according to the manufacturer's protocol. Four microlitres of total RNA was reverse transcribed using Thermoscript (Invitrogen, UK) with the poly-dT oligonucleotide primer included in the kit. 2 µl of resulting cDNA was used as template in PCRs designed to amplify the variable regions of the light and heavy chains.

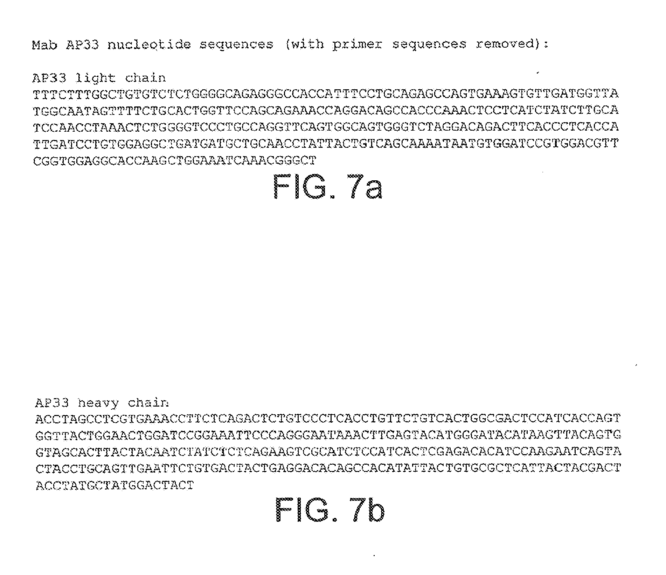

Heavy chain amplification was achieved as described previously (McCafferty & Johnson: Construction and Screening of Antibody Display Libraries. In: "Phage Display of Peptides and Proteins: A Laboratory Manual". Ed. Kay et al 1996, p79-111), using the sense primer VH1BACK (5'-AGG TSM ARC TGC AGS AGT CWG G-3') with antisense primer VH1FoR-2 (5'-GGG GCC AAG GGA CCA CGG TCA CCG TCT CCT CA-3') and HotStar Taq (Qiagen). Light chain amplification was achieved as described previously (Wang et al, 2000 J. Immunol. Methods 233, 167-177) using the primers Mk (5'-GGG AGC TCG AYA TTG TGM TSA CMC ARW CTA MCA-3') with reverse primer Kc (5'-GGT GCA TGC GGA TAC AGT TGG TGC AGC ATC-3'). PCR products were column purified using the Qiaquick PCR purification kit (Qiagen) then cloned into the Promega pGEM®-T vector, using the pGEM®-T vector system according to the manufacturer's recommendations. Two clones for each heavy and light chain were sequenced using the T7 forward and M13 reverse primer (Promega) and the ABI PRISM BigDye Terminator Cycle Sequencing Ready Reaction Kit (Perkin Elmer Applied Biosystems), according to the manufacturer's protocol. The determined nucleotide sequence for AP33 is shown in Figure 7.

### Results

The deduced amino acid sequences corresponding to the light and heavy chain variable regions of MAbs AP33 and 3/11 were compared. The most striking feature of this comparison was that the heavy chain CDR3 for MAb AP33 contained ten, predominantly hydrophobic, amino acids. In contrast, the MAb 3/11 heavy chain CDR3 contained only 3 amino acids. IgBLAST search revealed that MAb AP33 heavy and light chains were most similar to the mouse VH-36-60, subgroup VH-1 (Accession number K01569) and VK-21-10 (Accession number K02160). Germ-line analysis of the 3/11 sequences was not possible due to the paucity of rat germ-line sequences available on the Genbank database.

### Example 8 ― Neutralisation of HCV

In this example, AP33 is shown to be capable of neutralising HCV virus.

**Generation of a chimeric HCV J6-JFH1 genomic cDNA.** The plasmid pJFH1 carrying the full-length HCV genotype 2a strain JFH1 cDNA downstream of the T7 RNA polymerase promoter was supplied to us by T. Wakita (Wakita et al., 2005 Nature Medicine 11, 791-796). To generate a chimeric construct, the nucleotide sequences encoding core, E1, E2, p7 and a N-terminal portion of NS2 of strain JFH1 cDNA were replaced with those from an another genotype 2a strain J6CF (Yanagi et al., 1999 Virology 262, 250-263). Infectious virus generated from the resultant chimeric J6-JFH1 construct, called J71, was used in the virus neutralisation assay described below.

**HCV J71 RNA transfection and virus production in cell culture.** The J71 construct was linearized by cleavage at a restriction enzyme site located immediately following the 3' end of the virus genomic cDNA. HCV J71 RNA was transcribed *in vitro* from the linearized construct using the MEGAscript High Yield Transcription kit (Ambion) as described by the manufacturer. Approximately 10 µg *of in vitro* synthesised J71 RNA was mixed with Huh-7 cells in a 0.4 cm Gene Pulser cuvette (Bio-Rad) and pulsed once at 960 µF and 270V using the GenePulser Xcell (Bio-Rad) electroporator. The transfected cells were immediately mixed with cell medium and seeded into 80 cm² flask and onto coverslips. Following incubation at 37°C for 4 d, medium from flask was collected, clarified by brief centrifugation to remove cell debris, filtered through 0.45 µm pore-sized membrane, and used to infect naive Huh-7 cells. Following incubation at 37°C for 4 d, the infected cells were found to contain viral antigens confirming the presence of infectious virus progeny in the medium collected from the electroporated cells. The electroporated cells on coverslips were fixed and the presence of viral proteins determined by indirect immunofluorescence.

The number of viral antigen positive cells was found to increase upon passaging the electroporated cells such that by passage 7 most of the cells harboured replicating viral genomes.

**HCV neutralisation assay.** The cell culture-produced HCV J71 virus was harvested from the medium of viral RNA-transfected Huh-7 cells at passage 10, clarified, filtered as described above, and used in virus neutralisation assay as described below. The medium containing the virus was mixed with 200, 40, 8, 1.6, 0.32, 0.064 µg/ml of purified MAb AP33 or 3/11 and incubated for 1 h at 37°C. Each virus-antibody mix was then serially diluted 10-fold in complete medium ranging from 10⁻¹ to 10⁻⁷. Each dilution was used to infect Huh-7 cells (6 wells per dilution) in a 48-well tissue culture dish and the cells incubated at 37°C for 3 h after which the inoculum was removed, the cells re-fed with fresh medium and incubated at 37°C for 4 d. The cells were then washed once with PBS, fixed with methanol, and probed for the viral NS5a using a sheep anti-NS5a antiserum (a kind gift of Mark Harris, University of Leeds) and the bound antibody detected using anti-sheep IgG-FITC conjugate (Molecular Probe). The wells were scored for the presence or absence of fluorescing cells, and the virus infectivity was determined as TCID₅₀ (tissue culture infectious dose) essentially as described by Lindenbach et al (2005) Science 309, 623-626.

### Discussion

Previous studies have determined the neutralising capacity of sera or antibodies against only a very limited range of HCV genotypes (5, 21, 30). The inventors have used the HCVpp assay to assess the capacity of a range of E2-specific antibodies and sera to neutralise HCVpps carrying E1E2 representative of all of the major genotypes 1 through 6.

Having established a panel of functional E1E2 clones representative of all the major genotypes, the inventors went on to assess the capacity of the MAb AP33 and of rabbit antisera raised against either the HVR1 region or the ectodomain portion of the E2 protein of the H77c strain to neutralise HCVpp entry. Differences in the neutralisation potency between rabbits immunised with the same immunogen were also evident, although the rabbit sera raised against HVR1 and the ectodomain of E2 were both capable of neutralising HCVpp incorporating H77c E1E2. However, both showed reduced neutralisation of HCVpp incorporating heterologous genotype 1a E1E2, and neither neutralised HCVpp containing E1E2 derived from other genotypes tested. The high degree of HVR1 genetic variability explains the poor cross-neutralisation observed, and this finding is in keeping with previous reports of the restricted neutralising capacity of natural HVR1-specific antibodies (76). Immunisation of rabbits with the E2 ectodomain also resulted in the induction of neutralising antibodies, but again these responses were highly strain specific. Peptide mapping and competition assays showed that the most potently neutralising serum (R646) recognised both conformational and linear determinants. Neutralisation could be inhibited by native but not denatured sE2, indicating that the neutralising antibodies most likely recognise conformational epitopes.

Previous work has shown that antibodies elicited following immunisation with HCV envelope glycoproteins can at least partially protect against homologous challenge (33, 59). Similarly, a number of broadly neutralising human monoclonal antibodies have been described, all of which recognise conformational epitopes (1, 9, 37, 38, 40, 42). These antibodies could have a future role in the treatment of HCV infection; they might also serve to define future vaccine candidates. However, studies with HIV-1 have shown that focussing the immune response on epitopes recognised by broadly neutralising antibodies is a significant challenge. In this context, the finding that AP33 potently neutralises the entry of HCVpp carrying highly divergent E1E2 is significant, particularly as its epitope is linear and highly conserved across different genotypes of HCV. The epitope recognised by AP33 has been mapped to residues 412-423 (exemplified by the sequence QLINTNGSWHIN) and carries one potential N-linked glycosylation site (52). It is interesting to note that HCVpp derived from one genotype 5 isolate (UKN5.14.4), although infectious, was not recognised (and therefore not neutralised) by the MAb AP33. This isolate had a 4 amino acid change (QLIQNGSSWHIN) in the E2 region corresponding to the AP33 epitope, with a well-conserved N-linked glycosylation site shifted -1 relative to that in the other isolates. Subsequent analysis of sequences deposited into the Genbank database has shown the AP33 epitope to be highly conserved. The average diversity of sequences compared to the prototype AP33 epitope was 4.7 % and the majority of variable amino acids were located at the N-terminus of the predicted epitope. Importantly, sequences similar to that present in UKN5.14.4 were not evident. The inventors' preliminary data show that reversion of one of the 4 variant amino acids to its conserved counterpart (i.e. Q to N) renders the UKN5.14.4 HCVpp non-infectious (while also remaining non-reactive to MAb AP33) (not shown), thus highlighting the possible importance of this region of E2 in infection. This observation together with the fact that the infectivity of UKN5.14.4 HCVpp is not affected by the N415Q and the -1 shift of the N-linked glycosylation sequence further indicates that this particular isolate may represent a neutralisation escape mutant.

In conclusion the inventors have shown that the epitope defined by the AP33 antibody is highly conserved across all the major genotypes and that this antibody is capable of broad neutralisation.

### References

1. Allander et al, Journal of General Virology. 81:2451-2459.
2. Alter et al, 1992. New England Journal of Medicine 327:1899-1905.
3. Anonymous. 1999. Global surveillance and control of hepatitis C. J Viral Hepat 6:35-47.
4. Barth et al, 2003. Journal of Biological Chemistry 278:41003-41012.
5. Bartosch et al, 2003. Proceedings of the National Academy of Sciences of the United States of America 100:14199-14204.
6. Bartosch et al, 2003. J Exp Med 197:633-42.
7. Bartosch et al, 2003. Journal of Biological Chemistry 278:41624-41630.
8. Bartosch et al, 2003. Journal of Biological Chemistry:M305289200.
9. Bugli et al, 2001. J. Virol. 75:9986-9990.
10. Bukh et al, 1995. Semin Liver Dis 15:41-63.
11. Cerino et al, 2001. Journal of Immunology 167:3878-3886.
12. Charloteaux et al, 2002. Journal of Virology 76:1944-1958.
13. Chayama et al, 2000. Hepatology 32:1138-1144.
14. Choukhi et al, 1999. Journal of General Virology 80:3099-3107.
15. Choukhi et al, 1998. Journal of Virology 72:3851-3858.
16. Clayton et al, 2002. Journal of Virology 76:7672-7682.
17. Coequerel et al, 2003. Journal of Virology 77:10677-10683.
18. Cocquerel et al, 1998. Journal of Virology 72:2183-2191.
19. Cooreman M. P., & E. M. E. Schoondermark-Van de Ven. 1996. Scandinavian Journal of Gastroenterology Supplement 31:106-115.
20. Cormier et al 2004. Proceedings of the National Academy of Sciences of the United States of America 101:7270-7274.
21. De Beeck et al 2004. Journal of Virology 78:2994-3002.
22. Dubuisson et al 2000. Journal of Biological Chemistry 275:30605-30609.
23. Dubuisson et al 1994. Journal of Virology 68:6147-6160.
24. Dubuisson et al 1996. Journal of Virology 70:778-786.
25. Duvet et al 2002. Glycobiology 12:95-101.
26. Farci et al 1994. Proceedings of the National Academy of Sciences of the United States of America 91:7792-7796.
27. Farci et al 1996. Proceedings of the National Academy of Sciences of the United States of America 93:15394-15399.
28. Farci et al 2002. Proceedings of the National Academy of Sciences of the United States of America 99:3081-3086.
29. Flint et al 2000. Journal of Virology. 74:702-709.
30. Flint et al 2004. J. Virol. 78:6875-6882.
31. Flint et al, 1999. J Virol 73:6235-44.
32. **Flint *et al,*** as above.
33. Forns et al 2000. Hepatology 32:618-625.
34. Fukumoto et al 1996. Hepatology 24:1351-1354.
35. Goffard, A. & J. Dubuisson. 2003. Glycosylation of hepatitis C virus envelope proteins. Biochimie 85:295-301.
36. Gruener et al 2001. Journal of Virology 75:5550-5558.
37. Habersetzer et al 1998. Virology 249:32-41.
38. Hadlock et al 2000. Journal of Virology. 74:10407-10416.
39. Hsu et al 2003. Proc Natl Acad Sci U S A 100:7271-6.
40. Ishii et al 1998. Hepatology 28:1117-1120.
41. Kato, N. 2000. Microbial & Comparative Genomics 5:129-151.
42. Keck et al 2004. J. Virol. 78:9224-9232.
43. Lavillette et al. 2005. Hepatology 41:265-274.
44. Logvinoff et al 2004. Proc. Natl. Acad. Sci., USA 101:10149-10154.
45. Makris et al 1996. Journal of Haematology 94:746-752.
46. McKeating et al 2004. J. Virol. 78:8496-8505.
47. Meunier et al 1999. Virology 80:887-896.
48. Mizokami et al 1994. Journal of Hepatology 21:884-886.
49. Nakabayashi et al 1982. Cancer Res 42:3858-63.
50. Neumann et al 1998. Science 282:103-107.
51. Op De Beeck et al 2004. J Virol 78:2994-3002.
52. Owsianka et al 2001. Journal of General Virology. 82:1877-1883.
53. Patel et al 1996. Virology 217:111-23.
54. Patel et al 2000. Journal of General Virology. 81:2873-2883.
55. Pileri et al 1998. Science 282:938-411.
56. Poignard et al 1999. Immunity 10:431-8.
57. Polyak et al 2000. Forum 10:46-58.
58. Pozzetto et al 1996. Nephrology Dialysis Transplantation 11:2-5.
59. Puig et al 2004. Vaccine 22:991-1000.
60. Roccasecca et al 2001. Molecular Immunology 38:485-492.
61. Rosa et al, 1996. Proceedings of the National Academy of Sciences of the United States of America 93:1759-1763.
62. **Rosa *et al,*** as 61 above.
63. Scarselli et al 2002. Embo J 21:5017-25.
64. Simmonds et al 1994. Hepatology 19:1321-1324.
65. Simmonds et al 1990. J Virol 64:864-72.
66. Simmonds et al 1990. Lancet 336:1469-1472.
67. Slater-Handshy et al 2004. Virology 319:36-48.
68. Steinmann et al 2004. J. Virol. 78:9030-9040.
69. Triyatni et al 2002. Virology. 298:124-132.
70. Yanagi et al 1997. Proc Natl Acad Sci U S A 94:8738-43.
71. Zhang et al 2004. Journal of Virology 78:1448-1455.
72. Zhu, F., & D. D. Eckels. 2002. Human Immunology 63:710-718.
73. Zibert et al 1997. Journal of Virology 71:4123-4127.
74. Zibert et al 1999. Journal of Hepatology 30:177-184.
75. Zucchelli et al 2001. Hepatology 33:692-703.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

The present invention comprises the subject matter of the following numbered paragraphs:
1. Use of a ligand capable of binding to an epitope of the HCV E2 polypeptide defined by monoclonal antibody AP33 in the manufacture of a composition for the prophylaxis or treatment of infection by members of each of genotypes 1 to 6 of HCV.
2. Use according to numbered paragraph 1, wherein the ligand is capable of binding to a polypeptide epitope which has the sequence X₁LX₂NX₃X₄GX₅WX₆X, wherein X₁₋₇ is any amino acid.
3. Use according to numbered paragraph 2, wherein X₁ is selected from the group consisting ofS,E,Q,H,PandL.
4. Use according to numbered paragraph 2, wherein X₂ is selected from the group consisting of V, I, A, R and F.
5. Use according to numbered paragraph 2, wherein X₃ is selected form the group consisting ofS,T,H,LorA.
6. Use according to numbered paragraph 2, wherein X₄ is selected form the group consisting of N, Q or G.
7. Use according to numbered paragraph 2, wherein X₅ is selected form the group consisting ofS,KorT.
8. Use according to numbered paragraph 2, wherein X₆ is selected form the group consisting of H, r or Q.
9. Use according to numbered paragraph 2, wherein X₇ is selected form the group consisting of I, L, F or P.
10. Use according to numbered paragraph 2, wherein the polypeptide epitope is selected from the group consisting of QLINTNGSWHI, QLVNTNGSWHI, QLINSNGSWHI, SLINTNGSWHI, ELINTNGSWHI, HLANHQGKWRL, PLFNANGTWQF and ELRNLGGTWRP.
11. Use according to numbered paragraph 1, wherein the ligand is an immunoglobulin.
12. Use according to numbered paragraph 11, wherein the immunoglobulin is an immnuglobulin fragment selected from the group conssiting of Fab, F(ab')₂, Fv, scFv and single domain antibody (dAb) molecules.
13. Use according to numbered paragraph 11 or numbered paragraph 12, wherein the ligand comprises one or more CDRs derived from monoclonal antibody AP33.
14. Use according to numbered paragraph 13, wherein said one ore more CDRs is selected from the group consisting of:
   (a) RASESVDGYGNSFLH, LASNLNS, QQNNVDPWT, GDSITSGYWN, YISYSGSTY or ITTTTYAMDY;
   (b) sequences having one, two or three amino acid additions, substitutions or deletions from the sequences set forth in (a); and
   (c) sequences structurally similar to the sequences set forth in (a) when present in an immunoglobulin.
15. Use according to any one of numbered paragraphs 11 to 14, wherein the immunoglobulin is a humanised, veneered, resurfaced, CDR-grafted, SDR-transferred or deimmunised immunoglobulin.
16. Use according to any preceding numbered paragraph, for the prevention of the infection of a vertebrate cell by HCV.
17. A method for the prophylaxis or treatment of infection by two or more of genotypes 1-6 of HCV, comprising administering an effective amount of a ligand as defined in any one of numbered paragraphs 1 to 16.
18. A method for the prophylaxis or treatment of infection by two or more of genotypes 1-6 of HCV, comprising administering an effective amount of an immunoglobulin as defined in any one of numbered paragraphs 11 to 16.
19. An immunoglobulin molecule which neutralises HCV isolates belonging to two or more of genotypes 1-6 of HCV, wherein said immunoglobulin comprises one or more CDRs derived from monoclonal antibody AP33, and said immunoglobulin molecule is an immunoglobulin other than the monoclonal antibody AP33.
20. A immunoglobulin molecule according to numbered paragraph 19, wherein said one ore more CDRs is selected from the group consisting of:
   (a) RASESVDGYGNSFLH, LASNLNS, QQNNVDPWT, GDSITSGYWN, YISYSGSTY or ITTTTYAMDY;
   (b) sequences having one, two or three amino acid additions, substitutions or deletions from the sequences set forth in (a); and
   (c) sequences structurally similar to the sequences set forth in (a) when present in an immunoglobulin.
21. An immunoglobulin molecule which neutralises HCV isolates belonging to two or more of genotypes 1-6 of HCV and is capable of binding to a polypeptide epitope which has the sequence X₁LX₂NX₃X₄GX_{S}WX₆X₇, wherein X₁₋₇ is any amino acid, said immunoglobulin being other than monoclonal antibody AP33.
22. An immunoglobulin molecule according to any one of numbered paragraphs 19 to 21 comprising one or more human frameworks.
23. An immunoglobulin according to any one of numbered paragraphs 19 to 22, which is a humanised, veneered, resurfaced, CDR-grafted, SDR-transferred or deimmunised immunoglobulin.
24. An immunoglobulin according to any one of numbered paragraphs 19 to 23 comprising one or more human CDRs.
25. A polynucleotide encoding a polypeptide according to any one of numbered paragraphs 19 to 24.
26. A composition for inducing antibodies which bind to Hepatitis C Virus (HCV) E2 glycoprotein, the composition comprising a peptide having the amino acid residue sequence XLXNXXGXWXX and a physiologically acceptable carrier, excipient or diluent; the peptide optionally comprising additional amino acid residues at the N and/or C terminal but wherein the peptide does not encompass the entire HCV E2 glycoprotein nor the E2₆₆₀ fragment thereof (i.e. residues 384-660 of the HCV polyprotein), and wherein one or more of the amino acid residues may be covalently modified.
27. A composition according to numbered paragraph 26, wherein the peptide comprises the amino acid sequence X₁LX₂NX₃X₄GX₅WX₆X₇, wherein X₁ is selected from the group consisting of S, E, Q, H, P and L; X₂ is selected from the group consisting of V, I, A, R and F; X₃ is selected form the group consisting of S, T, H, L and A; X₄ is selected form the group consisting of N, Q and G; X₅ is selected form the group consisting of S, K and T; X₆ is selected form the group consisting of H, r and Q; and X₇ is selected form the group consisting of I, L, F and P.
28. A composition according to numbered paragraph 27, wherein the peptide comprises an amino acid sequence selected from the group consisting of: QLINTNGSWHI, QLVNTNGSWHI, QLINSNGSWHI, SLINTNGSWHI, ELINTNGSWHI, HLANHQGKWRL, PLFNANGTWQF and ELRNLGGTWRP.
29. A composition according to any one of numbered paragraphs 25-28, wherein the peptide additionally comprises a T cell epitope and/or a further B cell epitope.
30. A composition according to any one of numbered paragraphs 25-29, wherein the peptide comprises one or more repeats of an amino acid residue sequence in accordance with the general formula XLXNXXGXWXX.
31. composition according to numbered paragraph 30, wherein the peptide comprises two or more non-identical amino acid residue sequences in accordance with the formula XLXNXXGXWXX.
32. A composition according to any one of numbered paragraphs 25-31, wherein the peptide is present as part of a fusion protein.
33. A composition according to any one of numbered paragraphs 25-32, wherein the composition comprises a branched peptide.
34. A composition according to any one of numbered paragraphs 25-33, further comprising an adjuvant.
35. A nucleic acid construct encoding a peptide for use in a composition in accordance with any one of numbered paragraphs 25-33.
36. A kit for detecting the presence of HCV belonging to two or more of genotypes 1-6 of HCV, wherein said kit comprises an immunoglobulin which comprises one or more CDRs derived from monoclonal antibody AP33.
37. A kit according to numbered paragraph 36, wherein said one ore more CDRs is selected from the group consisting of:
   (a) RASESVDGYGNSFLH, LASNLNS, QQNNVDPWT, GDSITSGYWN, YISYSGSTY or ITTTTYAMDY;
   (b) sequences having one, two or three amino acid additions, substitutions or deletions from the sequences set forth in (a); and
   (c) sequences structurally similar to the sequences set forth in (a) when present in an immunoglobulin.

## Claims

1. Use of a ligand capable of binding to an epitope of the HCV E2 polypeptide defined by monoclonal antibody AP33 in the manufacture of a composition for the prophylaxis or treatment of infection by members of each of genotypes 1 to 6 of HCV.

2. Use according to claim 1, wherein the ligand is capable of binding to a polypeptide epitope which has the sequence X₁LX₂NX₃X₄GX₅WX₆X, wherein X₁₋₇is any amino acid.

3. Use according to claim 2, wherein the polypeptide epitope is selected from the group consisting of QLINTNGSWHI, QLVNTNGSWHI, QLINSNGSWHI, SLINTNGSWHI, ELINTNGSWHI, HLANHQGKWRL, PLFNANGTWQF and ELRNLGGTWRP.

4. Use according to claim 1, wherein the ligand is an immunoglobulin.

5. Use according to claim 4, wherein the ligand comprises one or more CDRs derived from monoclonal antibody AP33.

6. Use according to claim 5, wherein said one or more CDRs is selected from the group consisting of:
(a) RASESVDGYGNSFLH, LASNLNS, QQNNVDPWT, GDSITSGYWN, YISYSGSTY or ITTTTYAIVIDY;
(b) sequences having one, two or three amino acid additions, substitutions or deletions from the sequences set forth in (a); and
(c) sequences structurally similar to the sequences set forth in (a) when present in an immunoglobulin.

7. Use according to any preceding claims, for the prevention of the infection of a vertebrate cell by HCV.

8. A ligand as defined in any one of claims 1 to 7 for use in the prophylaxis or treatment of infection by members of each of genotypes 1 to 6 of HCV.

9. An immunoglobulin molecule which neutralises HCV isolates belonging to two or more of genotypes 1-6 of HCV, wherein said immunoglobulin comprises one or more CDRs derived from monoclonal antibody AP33, and said immunoglobulin molecule is an immunoglobulin other than the monoclonal antibody AP33.

10. The immunoglobulin molecule according to claim 9, wherein said one or more CDRs is selected from the group consisting of:
(a) RASESVDGYGNSFLH, LASNLNS, QQNNVDPWT, GDSITSGYWN, YISYSGSTY or ITTTTYAMDY;
(b) sequences having one, two or three amino acid additions, substitutions or deletions from the sequences set forth in (a); and
(c) sequences structurally similar to the sequences set forth in (a) when present in an immunoglobulin.

11. An immunoglobulin molecule which neutralises HCV isolates belonging to two or more of genotypes 1-6 of HCV and is capable of binding to a polypeptide epitope which has the sequence X₁LX₂NX₃X₄GX₅WX₆X₇, wherein X₁₋₇ is any amino acid, said immunoglobulin being other than monoclonal antibody AP33.

12. A composition for inducing antibodies which bind to Hepatitis C Virus (HCV) E2 glycoprotein, the composition comprising a peptide having the amino acid residue sequence XLXNXXGXWXX and a physiologically acceptable carrier, excipient or diluent; the peptide optionally comprising additional amino acid residues at the N and/or C terminal but wherein the peptide does not encompass the entire HCV E2 glycoprotein nor the E2₆₆₀ fragment thereof (i.e. residues 384-660 of the HCV polyprotein), and wherein one or more of the amino acid residues may be covalently modified.

13. The composition according to claim 12, wherein the peptide comprises the amino acid sequence X₁LX₂NX₃X₄GX₅WX₆X₇, wherein X₁ is selected from the group consisting of S, E, Q, H, P and L; X₂ is selected from the group consisting of V, I, A, R and F; X₃ is selected form the group consisting of S, T, H, L and A; X₄ is selected form the group consisting ofN, Q and G; X₅ is selected form the group consisting of S, K and T; X₆ is selected form the group consisting of H, r and Q; and X₇ is selected form the group consisting of I, L, F and P.

14. The composition according to any one of claims 12-13, wherein the peptide additionally comprises a T cell epitope and/or a further B cell epitope.

15. A kit for detecting the presence of HCV belonging to two or more of genotypes 1-6 of HCV, wherein said kit comprises an immunoglobulin which comprises one or more CDRs derived from monoclonal antibody AP33.
